(19) **Europäisches Patentamt / European Patent Office / Office européen des brevets**

(11) **EP 3 207 373 B1**

(12) # FASCICULE DE BREVET EUROPEEN

(45) Date de publication et mention
de la délivrance du brevet:
**27.02.2019 Bulletin 2019/09**

(21) Numéro de dépôt: **15785075.1**

(22) Date de dépôt: **15.10.2015**

(51) Int Cl.:
*C12Q 1/6844* (2018.01)     *G01N 33/543* (2006.01)
*B01L 3/00* (2006.01)     *G01N 33/68* (2006.01)
*G01N 35/08* (2006.01)     *G01N 15/00* (2006.01)

(86) Numéro de dépôt international:
**PCT/EP2015/073942**

(87) Numéro de publication internationale:
**WO 2016/059182 (21.04.2016 Gazette 2016/16)**

(54) **PROCÉDÉ D'ANALYSE DU CONTENU DE GOUTTES ET APPAREIL ASSOCIÉ**

VERFAHREN ZUR ANALYSE DES INHALTS VON TROPFEN UND ZUGEHÖRIGE VORRICHTUNG

METHOD OF ANALYSING THE CONTENT OF DROPS AND ASSOCIATED APPARATUS

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **15.10.2014 FR 1459903**

(43) Date de publication de la demande:
**23.08.2017 Bulletin 2017/34**

(73) Titulaire: **Ecole Supérieure de Physique et de
Chimie
Industrielles de la Ville de Paris
75005 Paris (FR)**

(72) Inventeurs:
• **GRIFFITHS, Andrew David
75005 Paris (FR)**
• **DOINEAU, Raphaël Clément Li-Ming
F-75005 Paris (FR)**
• **NIZAK, Clément
F-75011 Paris (FR)**
• **NGHE, Philippe Chi-Thanh
F-94160 Saint-mande (FR)**
• **BAUDRY, Jean Marie Pierre
F-75011 Paris (FR)**
• **BRIENT-LITZLER, Elodie Michèle Christine
F-78000 Versailles (FR)**

• **GODINA, Alexei
92600 Asnières-sur-Seine (FR)**

(74) Mandataire: **Lavoix
2, place d'Estienne d'Orves
75441 Paris Cedex 09 (FR)**

(56) Documents cités:
**WO-A1-2013/041983**

• **BRUNO TESTE ET AL: "A low cost and high
throughput magnetic bead-based
immuno-agglutination assay in confined
droplets", LAB ON A CHIP, vol. 13, no. 12, 26 mars
2013 (2013-03-26) , pages 2344-2349,
XP055202306, ISSN: 1473-0197, DOI:
10.1039/c3lc50353d**
• **TSUCHIYA ET AL: "On-chip polymerase chain
reaction microdevice employing a magnetic
droplet-manipulation system", SENSORS AND
ACTUATORS B: CHEMICAL: INTERNATIONAL
JOURNAL DEVOTED TO RESEARCH AND
DEVELOPMENT OF PHYSICAL AND CHEMICAL
TRANSDUCERS, ELSEVIER S.A, CH, vol. 130, no.
2, 18 octobre 2007 (2007-10-18), pages 583-588,
XP022550371, ISSN: 0925-4005**

EP 3 207 373 B1

**Description**

La présente invention concerne un procédé d'analyse du contenu de gouttes comprenant l'étape suivante :

- fourniture d'une pluralité de gouttes contenues dans un fluide porteur, au moins une des gouttes comprenant au moins un agrégat de particules définissant un objet allongé selon un axe principal, au moins certaines gouttes contenant au moins un élément cible propre à se fixer sur l'agrégat.

WO 2013/041983 A1 décrit un procédé de manipulation de particules magnétiques.

Un tel procédé est destiné par exemple à effectuer un criblage de molécules d'intérêts dispersées dans les gouttes. En particulier, le procédé est destiné à déterminer voire à sélectionner des gouttes comprenant un élément cible particulier, cet élément cible pouvant résulter d'une réaction chimique ou d'une réaction biologique.

En particulier, la mesure puis la sélection des gouttes peut être basée sur la concentration ou l'activité de liaison d'un produit.

Le document WO 2009/011808 A1 décrit un procédé de détermination d'une activité de fixation d'une protéine au sein d'une goutte.

La publication « Single-cell analysis and sorting using droplet-based microfluidics », de Mazutis et al. publiée en ligne, le 4 avril 2013 dans la revue Nature Protocols illustre ce principe.

Un hybridome de souris est encapsulé dans une goutte avec une bille recouverte d'anticorps anti-souris. L'hybridome sécrète des anticorps. Un anticorps secondaire couplé à un fluorophore permet de révéler la présence de l'anticorps sécrété. La distribution de l'anticorps secondaire est, en l'absence d'anticorps sécrété, homogène dans la goutte, mais elle se relocalise sur la bille en présence d'anticorps.

Le procédé est donc très sélectif pour déterminer l'activité d'une cellule particulière.

Par contre, un tel procédé présente divers inconvénients. Le procédé de compartimentation des cellules et des billes est aléatoire. Le nombre de billes dans les gouttes peut être estimé par une loi de distribution de Poisson. De même, le nombre de cellules au sein des gouttes peut être estimé par une loi de distribution de Poisson indépendante. Les concentrations initiales en billes et en gouttes sont ajustées pour avoir en moyenne une cellule et une bille par goutte. Seule une partie des gouttes présente donc un intérêt pour l'analyse réalisée.

Par ailleurs, la présence d'une bille unique de taille significative par goutte n'est pas favorable à la résolution de la méthode. En effet, les anticorps secondaires se répartissent sur toute la surface de la bille. La gamme dynamique du procédé est donc limitée par la surface externe disponible par bille.

Un but de l'invention est de fournir un procédé d'analyse plus fiable et plus sensible que les procédés existants.

A cet effet, l'invention a pour objet un procédé selon la revendication 1.

Le procédé selon l'invention peut comprendre l'une ou plusieurs des caractéristiques des revendications 2 à 15 ou des caractéristiques suivantes, prises isolément ou suivant toutes combinaisons techniquement possibles :

- les particules sont des particules magnétiques, avantageusement paramagnétiques ;
- la mesure d'un paramètre physique est une mesure de fluorescence ;
- Au moins une des gouttes comprend une cellule apte à sécréter l'élément cible et le procédé comprend une étape d'incubation au cours de laquelle l'élément cible est sécrété dans la goutte par la cellule ;
- Au moins une des gouttes comprend une cellule et le procédé comprend une étape de lyse cellulaire ;
- Au moins une des gouttes comprend un système de traduction in vitro apte à exprimer l'élément cible ;
- le procédé comprend une étape de mesure d'un paramètre physique, localement en un premier point situé à l'écart de l'agrégat dans au moins une des gouttes et du même paramètre physique, localement en un deuxième point au voisinage de l'agrégat dans la même goutte ;
- la dimension maximale des particules est inférieure à 50 % du diamètre de la goutte ;
- la goutte contient au moins une entité de signalisation, et la mesure du paramètre physique dépend de la position de l'entité de signalisation au sein de la goutte ou par rapport à l'agrégat ;
- l'entité productrice produit plusieurs éléments cibles choisis dans le groupe constitué par une protéine, un peptide, un morceau d'ADN ou d'ARN, un métabolite, un ion, un lipide et une biomolécule susceptible d'être produite par une cellule ;
- le procédé comporte une étape de détermination d'au moins une caractéristique de l'entité productrice ;
- l'étape de décision de classement a lieu après l'étape de mesure ;
- la goutte contient des particules superparamagnétiques, la goutte ou la partie de la goutte est dirigée vers la zone de classement par un moyen de direction choisi parmi une force magnétique, un champ électrique, une diélectrophorèse, une électrocoalescence ou une onde acoustique de surface ;
- une partie de la goutte est extraite au moyen de la force magnétique, la partie extraite formant une goutte auxiliaire et contenant l'agrégat.

L'invention a également pour objet un appareil d'analyse du contenu de gouttes selon la revendication 16.

L'invention sera mieux comprise à la lecture de la description qui va suivre, donnée uniquement à titre d'exemple, et faite en se référant aux dessins annexés, sur lesquels :

- la figure 1 est une représentation schématique des éléments principaux d'un premier appareil d'analyse selon l'invention,
- la figure 2 est une représentation schématique d'une étape de procédé avec le premier appareil,
- les figures 3 à 6 sont des photographies d'une partie d'un deuxième appareil suivant l'invention lors de différentes étapes de procédé suivant l'invention,
- la figure 7 est une représentation schématique d'un troisième appareil selon l'invention,
- la figure 8 est une représentation schématique d'un quatrième appareil selon l'invention,
- la figure 9 est une représentation schématique d'une étape de procédé avec le premier appareil,
- la figure 10 illustre la détermination d'un coefficient de dissociation Kd par le procédé,
- les figures 11 et 12 représentent des dispositifs de générations de gouttes,
- les figures 13 et 14 représentent des ensembles d'espacement de gouttes et de mesure,
- les figures 15 et 16 représentent des ensembles de lecture en deux dimensions,
- les figures 17, 18 et 19 illustrent l'exemple 3,
- les figures 20 et 21 illustrent l'exemple 4,
- les figures 22 et 23 illustrent l'exemple 5,
- la figure 24 est une représentation schématique d'une goutte lors d'une étape de mise en oeuvre d'un procédé,
- la figure 25 illustre l'exemple 6,
- les figures 26, 27 et 28 illustrent l'exemple 7,
- les figures 29 et 30 illustrent l'exemple 8,
- les figures 31 et 32 illustrent l'exemple 9,
- les figures 33 et 34 illustrent l'exemple 10.

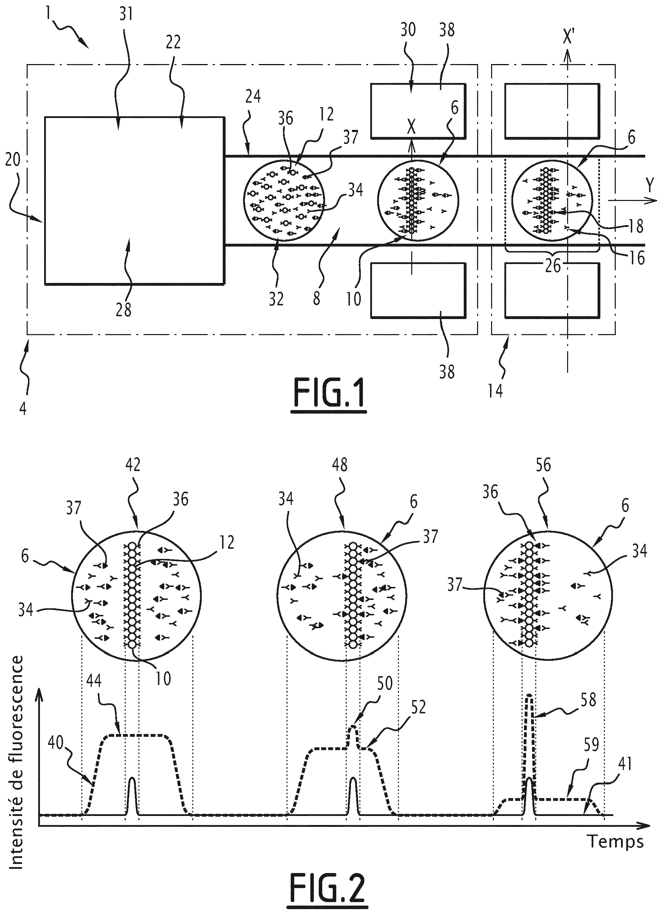

Un premier appareil d'analyse 1 du contenu de gouttes suivant l'invention est représenté sur la figure 1.

L'appareil 1 comprend un ensemble de fourniture 4 d'une pluralité de gouttes 6 contenues dans un fluide porteur 8, au moins une partie des gouttes 6 comprenant au moins un agrégat 10 de particules 12 définissant un objet allongé selon un axe principal X.

L'appareil 1 comprend, en outre, un ensemble de mesure 14 d'un paramètre physique dans la goutte.

L'ensemble de mesure 14 est, par exemple, propre à effectuer la mesure d'un paramètre physique, localement en un premier point 16 situé à l'écart de l'agrégat 10 dans au moins une des gouttes et du même paramètre physique localement en un deuxième point 18 au voisinage de l'agrégat 10 dans la même goutte.

L'appareil 1 comporte également un dispositif 20 comprenant un ensemble de mise en circulation 22, un conduit de circulation 24 et une zone de détection 26.

L'ensemble de mise en circulation 22 est apte à faire circuler chaque goutte 6 dans le fluide porteur 8 dans le conduit 24 sous forme d'un train de gouttes successives.

L'ensemble de fourniture 4 comprend un ensemble de chargement 28 et un ensemble d'agrégation 30. L'ensemble de fourniture 4 comprend en outre un ensemble d'espacement 31.

L'ensemble de chargement 28 est apte à fournir une pluralité de gouttes initiales 32 comprenant une dispersion de particules 12, au moins une des gouttes initiales 32 comprenant en outre au moins un élément cible 37.

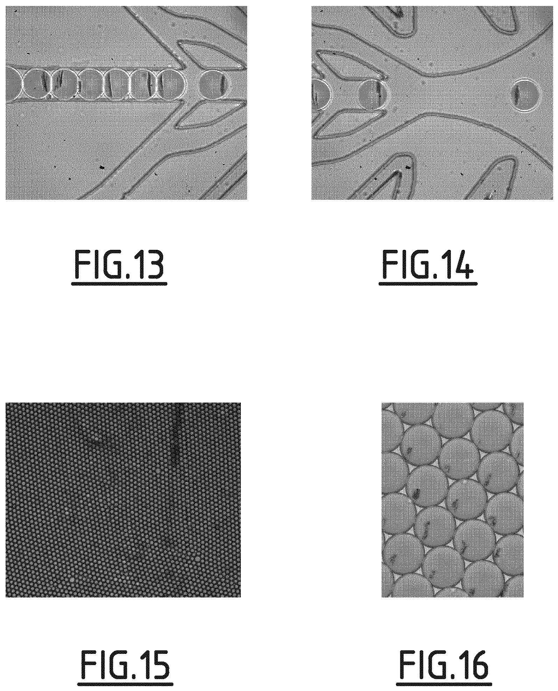

L'ensemble d'espacement 31 est apte à espacer deux gouttes successives 6 du train de goutte, c'est-à-dire à augmenter la distance entre deux gouttes successives. Par exemple, l'ensemble d'espacement 31 comporte une entrée de fluide porteur 8. Des exemples d'ensemble d'espacements sont représentés sur les figures 13 et 14.

Le fluide porteur 8 est apte à séparer deux gouttes successives 6 du train de gouttes pour empêcher leur contact. En variante, la séparation des gouttes 6 est effectuée par un dispositif mécanique.

Le fluide formant la phase interne des gouttes 6 et le fluide porteur 8 sont sensiblement immiscibles. Par exemple, les gouttes 6 comprennent une phase interne aqueuse et le fluide porteur 8 est une phase organique ou huileuse.

Le fluide porteur 8 est avantageusement une huile fluorée.

Le fluide porteur 8 ou le fluide formant la phase interne des gouttes comprend avantageusement un tensio-actif apte à empêcher la fusion de deux gouttes 6 en contact, comme par exemple tel que décrit dans le brevet US 2010/0105112 ou encore le surfactant EA de la société RainDance Technologies.

Par « sensiblement immiscibles », on entend généralement que la solubilité du fluide formant les gouttes dans le fluide porteur 8, mesurée à 25°C et à pression ambiante, est inférieure à 1 %.

La taille des gouttes 6 est, par exemple, comprise entre 1 $\mu$m et 1000 $\mu$m.

Le volume des gouttes 6 est compris avantageusement entre 0.1 picolitres et 1 microlitre.

Les gouttes 6 fournies sont sensiblement monodisperses. Ceci signifie que la polydispersité des gouttes 6 est inférieure à 5 %.

Dans l'exemple représenté, les gouttes 6 sont sphériques. En variante, les gouttes 6 sont de forme allongée selon l'axe de circulation Y du conduit 24. En variante, les gouttes 6 sont de forme de palet aplati selon un axe perpendiculaire à l'axe de circulation Y.

Chaque goutte initiale 32 comprend un fluide de base, une dispersion de particules 12 solides dans le fluide de base et une pluralité d'entités de signalisation 34. En outre au moins une goutte initiale 32 comprend au moins un élément cible 37.

Les particules 12 sont destinées à former l'agrégat 10 de forme allongée. Par exemple, les particules 12 sont des particules superparamagnétiques qui acquièrent un moment magnétique à l'application d'un champ magnétique. Le superparamagnétisme est un comportement des matériaux ferromagnétiques ou ferrimagnétiques qui apparaît lorsqu'ils sont sous la forme de petits grains ou nanoparticules. Dans des grains de taille suffisamment petite, l'aimantation peut se renverser spontanément sous l'influence de la température. Le terme « particules magnétiques » dans le texte désigne des particules superparamagnétiques.

Les particules magnétiques 12 sont, par exemple, choisies parmi les particules fournies par la société Dynal (Life Technologies) ou Ademtech ou Miltenyi.

Les particules 12 sont par exemple nanométriques. Ainsi, leur dimension maximale est inférieure à 1 $\mu$m et est par exemple comprise entre 50 nm et 1000 nm. Les particules 12 sont avantageusement sensiblement monodisperses. Par exemple, la variation entre les dimensions maximales des particules 12 est strictement inférieure à 10 %. La taille et le nombre de particules 12 par gouttes 6 sont choisies pour former le nombre désiré d'agrégat. La dimension maximale des particules 12 est inférieure à 50 % du diamètre de la goutte 6.

La concentration de particules 12 permet une stabilité colloïdale.

La concentration de particules 12 par gouttes 6 est telle que les particules 12 occupent entre 0,1 % et 5 % du volume de la goutte 6, par exemple 1,7 %.

Dans un exemple, chaque goutte 6 de 33 picolitres contient en moyenne 500 particules 12 de 300 nm de diamètres.

Les particules 12 forment initialement une dispersion homogène dans les gouttes initiales 32. Elles sont réparties sensiblement uniformément dans le volume de la goutte initiale 32. Ainsi, la concentration en particules 12 est homogène sur l'ensemble de la goutte initiale 32.

Les particules 12 possèdent avantageusement une surface permettant le couplage de molécules biologiques, constituée d'un matériau de surface. Par exemple les particules 12 sont couvertes d'un polymère présentant des fonctions COOH ou $NH_2$.

Avantageusement, ce matériau de surface permet également de limiter l'agrégation spontanée des particules 12 dans la goutte.

De manière additionnelle, il peut avantageusement favoriser la stabilité de l'agrégat 10, par exemple, via des liaisons non spécifiques entre le matériau d'une bille et de sa voisine dans l'agrégat.

Les particules 12 sont avantageusement fonctionnalisées. Ceci signifie notamment que le matériau de surface des particules 12 comporte des éléments fonctionnels.

Dans l'exemple représenté, les éléments fonctionnels comportent un élément de capture 36. L'élément de capture 36 est, par exemple, apte à capture l'élément cible 37. L'élément de capture 36 est apte à se lier indirectement à l'entité de signalisation 34 par l'intermédiaire de l'élément cible 37. En variante, l'élément de capture est apte à se lier directement à l'entité de signalisation 34.

Par exemple, l'élément de capture 36 sur les particules 12 est une protéine G, l'élément cible 37 est un anticorps, apte à se lier à la protéine G et l'entité de signalisation 34 est un antigène reconnu par l'anticorps, l'antigène étant apte à se lier à l'anticorps.

L'ensemble d'agrégation 30 est apte à engendrer une agrégation des particules 12 selon un axe principal X.

L'ensemble d'agrégation 30 comporte, par exemple, deux aimants 38 situés de part et d'autre du conduit 24. Le champ magnétique est non parallèle à l'axe de circulation Y et avantageusement perpendiculaire à l'axe de circulation Y. L'ensemble d'agrégation 30 permet la formation d'un agrégat allongé dans chaque goutte 6.

Dans un mode de réalisation les aimants 38 sont permanents.

En variante, l'ensemble d'agrégation 30 comporte un aimant non-permanent.

En variante, l'ensemble d'agrégation 30 est apte à basculer d'un mode actif à un mode inactif afin de générer des agrégats 10 allongés dans certaines gouttes seulement.

Chaque agrégat 10 de particules 12 comporte par exemple une colonne orientée suivant un axe principal X. La hauteur de la colonne est avantageusement comprise entre 50 % et 100 % du diamètre de la goutte 6. Sa largeur est, par exemple, inférieure à 60 % de sa hauteur.

De plus, l'ensemble d'agrégation 30 est, par exemple, apte à orienter l'agrégat suivant un axe préférentiel. Dans l'exemple représenté, l'axe X de l'agrégat 12 est perpendiculaire à l'axe de circulation Y des gouttes 6 dans le conduit de circulation 24.

L'ensemble de mesure 14 comprend par exemple une ligne laser apte à mesurer de manière optique l'intensité de la fluorescence selon une ligne s'étendant selon un axe X' perpendiculaire ou incliné par rapport à l'axe de circulation Y.

L'ensemble de mesure 14 est apte à effectuer la mesure au sein de la goutte dans la zone de détection 26.

L'axe X' de la ligne laser est avantageusement parallèle à l'axe de l'agrégat X dans la zone de détection 26.

Lorsque le débit du fluide porteur 8 est constant, la mesure en fonction du temps du signal obtenu par la ligne laser correspond à un balayage spatial de la goutte 6 passant devant la ligne laser. Ceci permet de prendre successivement plusieurs points de mesure et en particulier au moins un premier point 16 de mesure situé à l'écart de l'agrégat 10 et un deuxième point 18 de mesure situé plus proche de l'agrégat 10, au voisinage de l'agrégat 10.

En pratique, une pluralité de points de mesure successifs sont pris sur toute la dimension longitudinale de la goute 6 lors de son passage progressif en regard de l'ensemble de mesure 14.

Dans l'exemple représenté sur les figures 1 et 2, l'entité de signalisation 34 est fluorescente.

Le conduit de circulation 24 est destiné à permettre la circulation des gouttes 6, 32 suivant l'axe de circulation Y dans un sens de circulation allant de l'ensemble de fourniture 4 vers l'ensemble de mesure 14.

Le conduit de circulation 24 présente avantageusement un diamètre intérieur inférieur ou égal à 1 mm.

Le conduit de circulation 24 est allongé selon l'axe de circulation Y. Le conduit de circulation 24 présente une section transversale intérieure de contour arrondi tel que circulaire ou elliptique, ou de contour polygonal tel que rectangulaire.

Le conduit de circulation 24 est par exemple défini dans un matériau translucide permettant la mesure de paramètres optiques par l'ensemble de mesure 14. En variante, le conduit de circulation 24 définit au moins une fenêtre de mesure transparente dans la zone de détection 26.

Les parois du conduit de circulation 24 sont étanches au fluide porteur 8.

Par exemple, le conduit de circulation 24 est défini dans un tube capillaire de dimension interne avantageusement inférieure à 1 mm. En variante, le conduit de circulation 24 est défini dans une puce microfluidique.

L'ensemble de mise en circulation des gouttes 22 est destiné à déplacer une à une les gouttes 6, 32 dans le conduit 24 dans le sens de circulation.

L'ensemble de mise en circulation 22 comprend par exemple un pousse-seringue permettant d'appliquer des débits contrôlés au fluide porteur 8. En variante l'ensemble de mise en circulation 22 comprend un contrôleur de pression.

Un premier procédé d'analyse suivant l'invention mis en oeuvre dans le premier appareil 1 va maintenant être décrit.

Un appareil 1 tel que précédemment décrit est fourni. Des gouttes initiales 32 telles que décrites plus haut sont préparées dans un fluide porteur 8.

De préférence, les particules 12 sont dispersées de manière homogène dans chaque goutte initiale 32. Compte tenu de la faible taille des particules 12 individuelles par rapport aux gouttes initiales 32, chaque goutte initiale 32 contient un nombre élevé de particules individuelles 12 par exemple supérieur à 10. La probabilité d'obtenir une goutte initiale 32 dépourvue de particules 12 est très faible, voire nulle.

De préférence, les éléments cibles 37 et les entités de signalisation 34 sont dispersés de manière homogène dans chaque goutte initiale 32.

Au sein de la goutte initiale 32, des liaisons se forment entre les éléments ayant des affinités particulières.

Dans un exemple, chaque élément cible 37 se lie à une entité de signalisation 34 et à un élément de capture 36. L'entité de signalisation 34 est ainsi relocalisée sur une particule 12.

Dans la suite, on entend par entités « relocalisées», les entités liées à l'agrégat 10.

Les gouttes initiales 32 sont mises en circulation conjointement avec le fluide porteur 8 dans le conduit 24 par l'ensemble de mise en circulation 22.

Au moins une goutte initiale 32 est conduite vers l'ensemble d'agrégation 30. Un agrégat 10 de particules 12 définissant un objet allongé selon un axe principal X est formé par l'ensemble d'agrégation 30 dans la goutte initiale 32.

De préférence, lorsque les particules 12 sont des particules magnétiques, elles s'alignent le long de l'axe principal X lors de leur passage en regard de chaque aimant 38 de l'ensemble d'agrégation 30.

La goutte 6 comprenant l'objet allongé est conduite vers la zone de détection 26. Un paramètre physique est mesuré localement par l'ensemble de mesure 14 en au moins un premier point 16 dans au moins une des gouttes 6.

Dans une mise en oeuvre particulière, un paramètre physique est mesuré localement par l'ensemble de mesure 14 en au moins un premier point 16 dans au moins une des gouttes 6 et le même paramètre physique est mesuré localement en au moins un deuxième point 18 au voisinage de l'agrégat 10 dans la même goutte 6 par l'ensemble de mesure 14.

La figure 2 représente à titre illustratif différentes mesures obtenues pour différentes gouttes 6. Le graphique représente l'intensité de fluorescence mesurée par la ligne laser en fonction du temps.

L'intensité de fluorescence est mesurée dans une gamme de longueur d'onde caractéristique de l'entité de signalisation 34. Dans l'exemple, l'intensité de fluorescence est, en outre, mesurée dans une gamme de longueur d'onde caractéristique des particules 12, les particules 12 étant fluorescentes. L'agrégat 10 est ainsi plus facilement repérable.

L'intensité de fluorescence 40 correspondant à la fluorescence de l'entité de signalisation 34 mesurée sur la ligne laser est présentée en pointillés sur la figure 2 pour différentes gouttes 6.

L'intensité de fluorescence 41 correspondant à la fluorescence des particules 12 est présentée en traits pleins sur la figure 2 pour différentes gouttes 6.

L'étape de mesure comporte la détermination du paramètre physique localement en une pluralité de points situés dans la goutte. Elle comprend en outre avantageusement un cumul des valeurs mesurées en une pluralité de points, par exemple la détermination de l'intégrale des valeurs mesurées au sein de la goutte 6.

La première goutte 42 représentée est une goutte 6 dans laquelle les différentes entités de signalisation 34 n'ont pas été relocalisées sur les particules 12. La répartition des entités de signalisation 34 est homogène au sein de la goutte 6. Un signal d'intensité de fluorescence sous forme d'un plateau 44 est mesuré.

La deuxième goutte 48 représentée est une goutte 6 dans laquelle une partie des entités de signalisation 34 a été relocalisé sur les particules 12. En effet, ces entités de signalisation 34 sont liées à un élément cible 37 capturé par l'élément de capture 36. L'intensité de fluorescence au voisinage de l'agrégat 10 est donc plus importante que dans le reste de la goutte 6. Un signal d'intensité de fluorescence présentant un pic 50 en plus d'un plateau 52 est mesuré.

La hauteur du plateau 52 de la deuxième goutte 48 est plus faible que la hauteur du plateau 44 de la première goutte 42 car moins d'entités de signalisations 34 sont libres à l'écart de l'agrégat 10.

La troisième goutte 56 représentée est une goutte 6 dans laquelle une proportion plus importante des entités de signalisation 34 a été relocalisée sur l'agrégat. Un signal d'intensité de fluorescence présentant un pic 58 et un plateau 59. La hauteur du pic 58 mesurée est plus importante que la hauteur du pic 50 mesurée dans la deuxième goutte 48 car plus d'entités de signalisation 34 sont capturées par les particules 12 et sont donc situées au voisinage de l'agrégat 10.

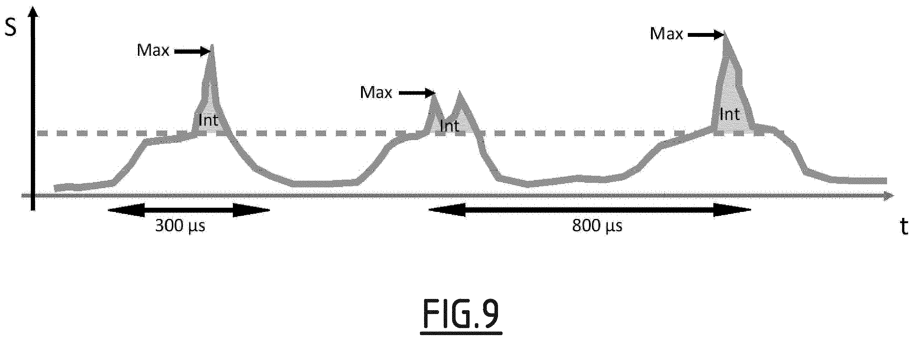

La figure 9 illustre le choix des paramètres utiles pour estimer la concentration d'entités de signalisation 34 relocalisées.

Sur la figure 9 qui représente le signal S au cours du temps t, on voit trois gouttes contenant un (à gauche et à droite) ou deux (agrégats) qui présentent un pic plus élevé de signal. Le paramètre utile peut-être le maximum du signal (indiqué Max) où l'intégrale du signal par rapport à un seuil donné (Int).

Une première méthode consiste à estimer cette concentration par la valeur maximale du signal (Max) dans chaque goutte 6, c'est-à-dire la hauteur des pics de signal relocalisés sur l'agrégat.

Une seconde méthode, plus précise, consiste à calculer l'intégrale des signaux (Int) pour chaque goutte 6 au-delà d'un seuil fixé par l'utilisateur, comme représenté par exemple sur la figure 9. Cette méthode peut s'avérer plus intéressante pour limiter la dispersion du signal, comme cela sera illustré dans l'exemple 3.

Ces deux méthodes de traitement du signal peuvent être effectuées en temps réel.

D'autres méthodes, par exemple combinant ces approches, pourraient être appliquées, par exemple pour mesurer à la fois l'entité de signalisation 34 relocalisée et non relocalisée.

L'invention permet, en outre, de mesurer la concentration de l'élément cible 37 dans la goutte 6.

Un cas simple pour ce faire est de se placer dans le cas où :

- l'élément de capture 36 est en quantité suffisante et d'affinité suffisante pour l'élément cible 37 pour capturer au moins plus de 90 % de l'élément cible sur l'agrégat, avantageusement la totalité ;
- la concentration de l'entité de signalisation 34 est supérieure à celle de l'élément cible 37 et la constante de dissociation Kd entre l'entité de signalisation 34 et l'élément cible 37 est inférieure à la concentration de l'élément cible 37, avantageusement d'un facteur supérieur à 10. Ceci est typiquement le cas quand on utilise des réactifs de dosage optimisés comme des anticorps monoclonaux de Kd subnanomolaires, et qu'on veut détecter des concentrations d'élément cible 37 supérieures au nanomolaire, comme illustré dans l'Exemple 5.

On entend par « nanomolaire » égal à 1 nanomol/L.

Dans ces conditions particulière, la présence de chaque élément cible 37 donne lieu à la formation d'un complexe élément de capture 36 - élément cible 37 - entité de signalisation 34. La concentration de l'élément cible 37 est donc proportionnelle au signal de l'entité de signalisation 34 relocalisé sur l'agrégat 10. D'autres conditions permettent de réaliser cette quantification et seront évidentes à l'homme de l'art en modifiant les concentrations et les affinités des éléments de capture 36, ou des entités de signalisation 34 pour l'élément cible 37.

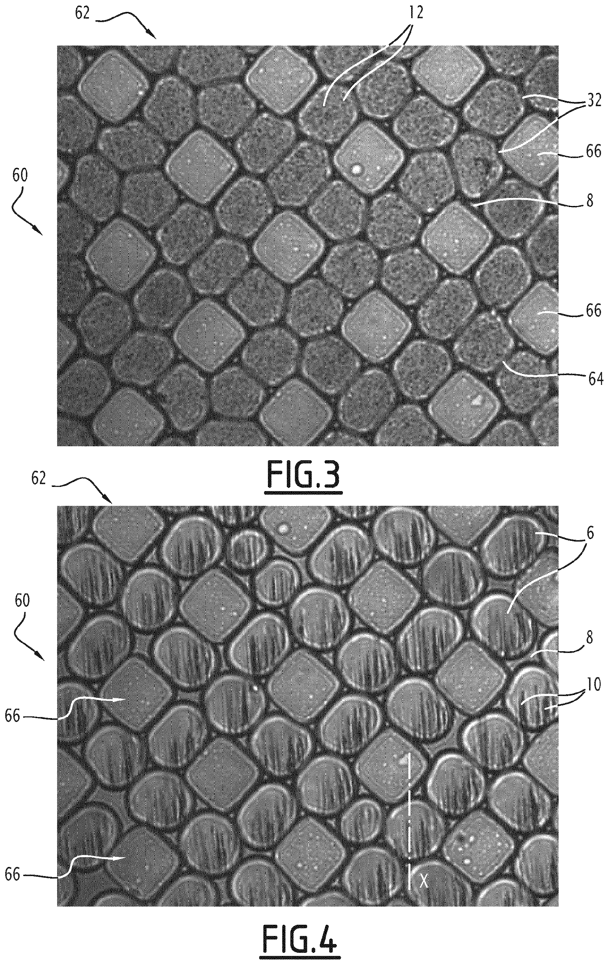

Les figures 3 à 6 illustrent une partie d'un deuxième appareil 60 suivant l'invention.

Ce deuxième appareil 60 diffère du premier appareil 1 en ce que le dispositif 20 comporte une chambre 62. La chambre 62 comporte une pluralité de passages de circulation 64 et une pluralité de plots de séparation 66.

D'autres chambres sont possibles. Dans une variante, des chambres ne comprennent pas de plots de séparation tels que décrits dans le document PCT/FR2009/051396 et illustré sur les figures 15 et 16.

La chambre 62 est destinée à stocker une pluralité de gouttes 6 dans un fluide porteur 8 pendant une étape d'agrégation ou une étape d'orientation et pendant l'étape de mesure.

L'ensemble de mesure du deuxième appareil 60 diffère de l'ensemble de mesure 14 du premier appareil 1 en ce qu'il est apte à mesurer le paramètre physique simultanément sur plusieurs gouttes 6 présentes dans la chambre 62.

La figure 3 présente la chambre 62 contenant des gouttes initiales 32 dans un fluide porteur 8. La dispersion des particules 12 magnétique est visible.

La figure 4 présente la même chambre 62 après la formation des agrégats 10 dans les gouttes. Une pluralité d'agrégats allongés est formée dans chaque goutte 6.

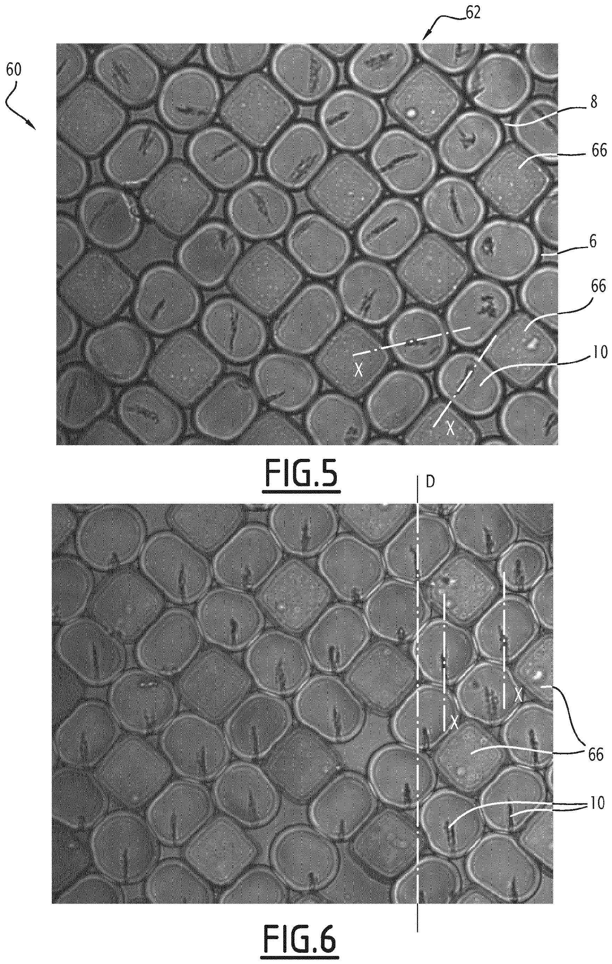

La figure 5 présente dans un même dispositif 60 une pluralité de gouttes 6 présentant des agrégats 10 allongés. La nature et la quantité de gouttes 6 ont été ajustées pour qu'un seul agrégat 10 allongé soit présent par goutte. La présence d'un unique agrégat 10 par goutte 6 facilite la mesure.

La figure 6 présente la même chambre 62 après une étape d'orientation des agrégats selon un même axe de détection D.

Le procédé d'analyse suivant l'invention à partir de ce deuxième appareil 60 diffère du procédé précédemment décrit en ce que la mesure est effectuée sur la pluralité de gouttes 6 simultanément, par exemple par mesure dans toute la chambre 62 en même temps, et non par circulation des gouttes 6 devant un détecteur.

Le procédé diffère également en ce qu'il comprend avant l'étape de mesure, une étape d'orientation de l'axe principal X de l'agrégat 10 selon un axe de détection D.

Avantageusement, on pourra multiplier les axes de détection, en appliquant des champs magnétiques d'orientation variable. Cette approche à l'avantage de permettre de discriminer l'agrégat 10 d'autres objets de la goutte non magnétiques, ou réduire les signaux parasites. Par exemple, le bruit de fond en fluorescence peut être réduit. Par exemple, la détection selon différents axes permet de distinguer une relocalisation de l'entité de signalisation 34 sur un agrégat 10 d'une relocalisation de l'entité de signalisation 34 sur un autre objet de la goutte 6, par exemple sur une cellule.

Une implémentation de cette idée consiste à appliquer un champ magnétique B1 pour aligner l'axe principal X de l'agrégat 10 selon une première orientation D1, puis à appliquer un champ magnétique B2 perpendiculaire à B1 pour aligner l'axe principal X de l'agrégat 10 selon une deuxième orientation D2 perpendiculaire à D1.

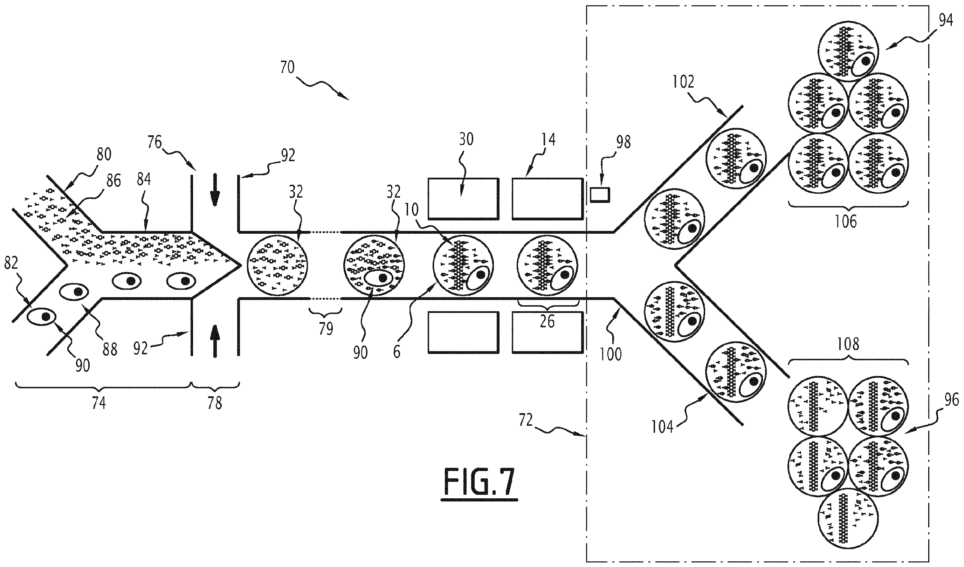

Un troisième appareil 70 suivant l'invention est représenté sur la figure 7.

Ce troisième appareil 70 diffère du premier appareil 1 en ce qu'il comporte en outre un ensemble de classement 72.

De plus, le troisième appareil 70 diffère du premier appareil 1 en ce que l'ensemble de chargement 28 comprend une zone d'entrée 74 de la phase interne et une zone d'entrée du fluide porteur 76 et une zone de jonction 78. L'ensemble de chargement 28 comprend en outre une zone d'incubation 79.

La zone d'entrée de la phase interne 74 comporte un premier conduit d'entrée 80, un deuxième conduit d'entrée 82 et un conduit de co-écoulement 84.

Le premier conduit d'entrée 80 est destiné à l'introduction de la première masse de fluide 86 destinée à former une partie de la phase interne des gouttes. Dans l'exemple, la première masse de fluide interne comporte les particules 12 et une pluralité d'entités de signalisation 34.

Le deuxième conduit d'entrée 82 est destiné à l'entrée de la deuxième masse de fluide 88 destinée à former une partie de la phase interne des gouttes. Dans l'exemple, la deuxième masse de fluide interne comporte une suspension d'entité productrices 90 de l'élément cible 37. Les entités productrices 90 sont par exemples des cellules 90.

Les cellules 90 sont avantageusement des cellules aptes à sécréter un élément cible 37. En particulier, certaines cellules 90 sont des cellules sécrétant des anticorps comme des hybridomes ou des plasmocytes. Les anticorps secrétés sont reconnus à la fois par les éléments de capture 36 des particules 12 et par les entités de signalisation 34.

La présence de ces éléments cibles 37 dans la goutte 6 permet la relocalisation des entités de signalisation 34 sur l'agrégat 10.

La concentration des cellules 90 dans la deuxième masse de fluide est avantageusement telle qu'une proportion importante de gouttes contiennent une cellule 90 seulement, par exemple plus de 10 % des gouttes contient une cellule 90.

Le conduit de co-écoulement 84 permet une répartition des deux masses de fluide 86, 88 destinée à former la phase interne.

La zone d'entrée du fluide porteur 76 est destinée à l'entrée du fluide porteur 8. Dans l'exemple représenté, le fluide porteur 8 entre par deux conduits d'entrées 92.

La zone de jonction 78 joint la zone d'entrée du fluide porteur 76 et la zone d'entrée de la phase interne 74. En particulier, la zone de jonction joint le conduit de co-écoulement 84 aux conduits d'entrées 92 du fluide porteur.

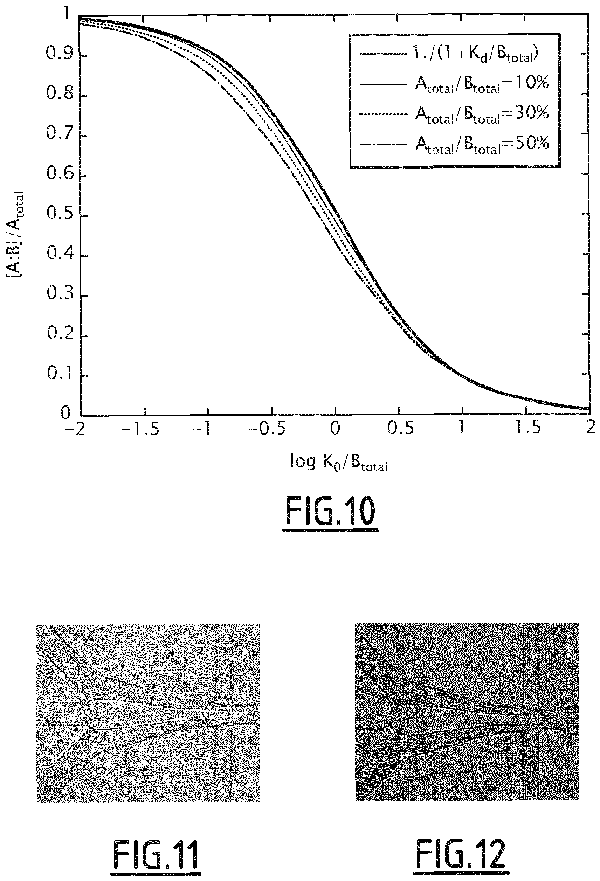

La zone de jonction 78 est apte à former les gouttes initiales 32. La zone de jonction 78 représentée ici est une jonction de focalisation hydrodynamique. Des exemples de jonctions de focalisation hydrodynamique sont illustrés sur les figures 11 et 12. En variante les gouttes initiales 32 sont formées dans une jonction T.

Les gouttes initiales 32 comprenant un mélange des deux masses de fluides 86, 88 sont formées. Les gouttes initiales 32 comprennent une dispersion de particules 12 et des entités de signalisation 34.

Au moins certaines gouttes initiales 32 comprennent en outre une cellule 90.

La zone d'incubation 79 est située en aval de la zone de jonction 78. La zone d'incubation est destinée à permettre la sécrétion de l'élément cible 37 par les cellules 90. En variante, les cellules 90 sont destinées à être lysées dans la zone d'incubation 79 de sorte que l'élément cible 37 soit libéré.

En variante, l'incubation est effectuée hors du dispositif 20.

Le troisième appareil 70 diffère également en ce que le dispositif 20 comprend en outre une pluralité de zones de classement 94, 96 et un moyen de direction 98 de la goutte ou une partie de la goutte sélectivement vers une zone de classement 94, 96.

Les zones de classement 94, 96 sont situées en aval de la zone de détection 26. Le conduit 24 comporte une bifurcation 100 en deux conduits de sorties 102, 104. La première zone de classement 94 comporte le premier conduit de sortie 102 destiné à recevoir un premier groupe de gouttes 106. La deuxième zone de classement 96 comporte le deuxième conduit de sortie 104 destiné à recevoir un deuxième groupe de gouttes 108. En variante, le dispositif 20 comporte un nombre plus important de zones de classement 94, 96 suivant le nombre de critères de tri.

Le moyen de direction 98 des gouttes sélectivement est par exemple apte à diriger une goutte 6 vers une zone de classement 94, 96 au moyen d'une force magnétique.

En variante les gouttes 6 sont dirigées au moyen d'électrodes.

Par exemple, les gouttes sont dirigées vers une zone de classement, par diélectrophorèse, par électrocoalescence avec un courant ou par des ondes acoustiques de surface (SAW).

Le procédé d'analyse avec le troisième appareil 70 suivant l'invention va maintenant être décrit.

Un appareil 70 tel que précédemment décrit est fourni. Une suspension de particules 12 magnétiques et d'entités de signalisation 34 est préparée et injectée dans le premier conduit d'entrée 80.

Une suspension de cellules 90 est préparée et injectée dans le deuxième conduit d'entrée 82.

Un fluide porteur 8 est fourni et injecté dans les conduits d'entrées de fluide porteur 92.

Les fluides 86, 88 sont mis en mouvement au moyen des ensembles de mise en circulation 22. Les gouttes initiales 32 sont formées dans la zone de jonction 78.

Le procédé comporte, en outre, une étape d'incubation au cours de laquelle la cellule 90 compartimentée sécrète l'élément cible 37, par exemple, la protéine à analyser.

En variante le procédé comporte une étape de lyse cellulaire permettant la libération de l'élément cible 37, par exemple, la protéine à analyser hors du cytoplasme.

Ces étapes de lyses ou d'incubation sont réalisées dans la zone d'incubation 79 du dispositif 20.

En variante, ces étapes sont réalisées hors du dispositif 20.

Les étapes de formation de l'agrégat 10 et de mesure sont les mêmes que pour le procédé d'analyse avec le premier appareil 1.

Le procédé diffère en ce que l'étape de mesure est suivie d'une étape d'analyse. L'étape d'analyse permet de déterminer à quel groupe 106, 108 appartient une goutte 6 suivant des critères prédéterminée et de générer une décision de classement par goutte 6 après l'étape de mesure.

Suivant la décision de classement, la goutte 6 est dirigée vers l'une des zones de classement 94, 96 par le moyen de direction 98.

Dans l'exemple représenté sur la figure 7, les gouttes 106 dans lequel le signal de forte intensité de fluorescence est localisé majoritairement au voisinage de l'agrégat 10 sont dirigées dans la première zone de classement 94. Les gouttes du premier groupe 106 correspondent par exemple aux troisièmes gouttes 56 de la figure 2.

Ces gouttes 106 contiennent par exemple les cellules 90 aptes à générer une protéine d'intérêt. Les gouttes 106 sont éventuellement récupérées pour que leur contenu soit analysé par d'autres techniques ou pour que les cellules 90 soient remises en culture.

Les gouttes 108 dans lequel un signal différent, notamment un signal sensiblement homogène sur la goutte 108, a été mesuré sont dirigées vers une autre zone de classement 96. Le deuxième groupe de gouttes 108 comporte par exemple des gouttes ne comprenant pas de cellules 90 et des gouttes contenant des cellules 90 ne produisant pas la protéine en quantité ou en qualité suffisante.

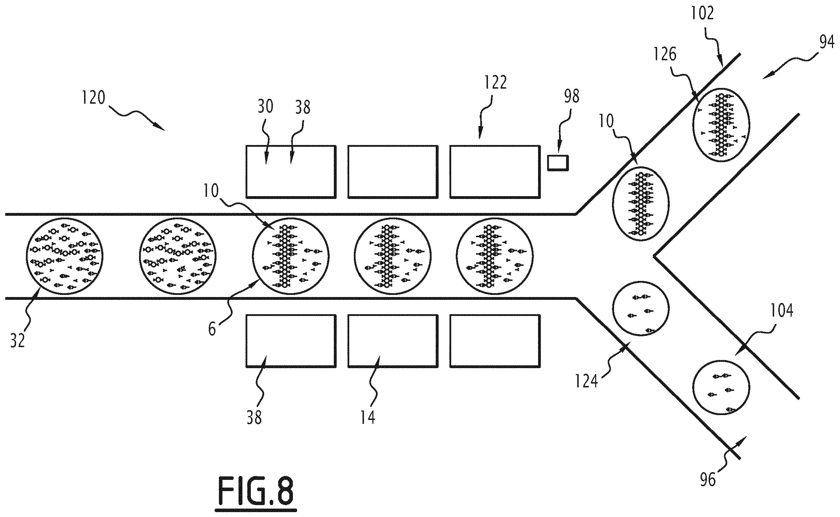

Un quatrième appareil 120 suivant l'invention est représenté sur la figure 8. Cet appareil 120 diffère du premier appareil 1 en ce qu'il comprend en outre un ensemble d'extraction 122.

L'ensemble d'extraction 122 est apte à séparer la goutte 6 en deux gouttes extraites 124, 126, une goutte primaire 124 et une goutte auxiliaire 126, la goutte auxiliaire 126 comprenant l'agrégat 10.

Avantageusement les particules 12 sont magnétiques et l'ensemble d'extraction 122 est apte à appliquer une force magnétique pour réaliser l'extraction.

En outre, le quatrième appareil 120 comprend en aval de l'ensemble d'extraction 122, un ensemble de classement 72 similaire à celui du troisième appareil 70 suivant l'invention permettant de séparer les gouttes auxiliaires 126 des gouttes primaires 124.

Dans une variante, les ensembles de classement et d'extraction sont les mêmes.

Un tel appareil 120 est utile notamment pour réaliser de la capture sélective au moyen des agrégats 10 allongés. Les éléments cibles 37 capturés par les particules 12 sont destinées à être élués après récupération des gouttes sélectionnées.

Un cinquième appareil suivant l'invention diffère du premier appareil en ce qu'il comporte un dispositif d'injection complémentaire et une deuxième zone de détection. Le dispositif d'injection complémentaire est apte à ajouter des éléments dans la goutte après la détection.

Le dispositif d'injection complémentaire est par exemple un dispositif de fusion de goutte ou un dispositif de pico-injection.

Le procédé d'analyse suivant l'invention à partir de ce cinquième appareil comporte une étape d'injection après l'étape de mesure, et une étape de mesure après l'étape d'injection.

Par exemple, l'entité de signalisation 34 est fluorescente et une entité supplémentaire non fluorescente est ajoutée lors de l'étape d'injection complémentaire. L'entité supplémentaire a les mêmes caractéristiques que l'entité de signalisation 34 excepté sa fluorescence. Par exemple, l'entité supplémentaire présente la même affinité pour l'élément cible 37 que l'entité de signalisation 34. Lors de la première étape de mesure, le taux de capture des entités de signalisation 34 fluorescentes est déterminé. Après l'injection, les entités supplémentaires et les entités de signalisation 34 sont en compétition pour se lier à l'élément cible 37 au voisinage de l'agrégat 10.

La deuxième mesure est effectuée après un certain temps et permet de déterminer le taux de décrochement des entités de signalisation 34 fluorescentes et donc de déterminer la constante de dissociation $k_{off}$ de la liaison entre l'entité de signalisation 34 et l'élément cible 37.

L'utilisation d'une dispersion de particules 12 de faibles tailles par rapport à la taille des gouttes assure une distribution homogène des particules 12 dans les gouttes 6, et donc la formation quasi certaine d'un agrégat 10 de taille significative dans chaque goutte 6.

Globalement, ce procédé permet de doser/quantifier un analyte dans la goutte. La formation d'un agrégat allongé 10 permet d'obtenir un meilleur rapport signal sur bruit et un intervalle dynamique plus important par rapport à l'essai décrit dans Mazutis et al. (Nat prot 2013) où une seule bille est encapsulée. En effet le signal généré par l'entité de signalisation 34 sera concentré sur une largeur plus petite que celle d'une sphère de surface égale. La hauteur du pic ainsi que présenté sur la Figure 2 sera donc plus élevée que dans le cas d'une seule bille pour un même nombre d'entité de signalisation 34 relocalisées.

Ce procédé est utilisable dans de nombreux procédés d'analyse biologique.

Le procédé selon l'invention peut s'appliquer à de nombreux types d'analytes, de biomolécules, de polypeptides, de protéines, de métabolites, d'acides nucléiques, de cellules, d'organelles, de microparticules et de nanoparticules, de polymères, de colloïdes, d'agents infectieux, de composés alimentaires, d'échantillons environnementaux.

L'appareil selon l'invention peut être intégré comme une brique technologique dans des dispositifs plus complexes en particulier dans un dispositif de criblage à haut débit, dans un laboratoire sur puce, dans un dispositif de « point of care » dans des instruments de laboratoires, des robots, ou autres.

En outre, le procédé selon l'invention peut être intégré dans des protocoles complexes permettant le diagnostic, la découverte de médicaments, la découverte de cibles, l'évaluation d'un médicament.

De plus les systèmes microfluidiques selon l'invention et les procédés selon l'invention peuvent être combinés ou inclus dans d'autres types de composants micro fluidiques ou pour d'autres fonctions micro fluidiques connues dans l'état de l'art.

En outre, en raison de la petite taille des échantillons utilisés, l'invention est particulièrement intéressante pour des applications dans des essais cellulaires, en particulier pour le criblage de cellules uniques et en biologie digitale.

Par digitale, on entend que les opérations biologiques sont réalisées sur une seule copie de l'objet chimique ou biologique et que le résultat de l'opération de chacune des copies uniques peut être isolé, en opposition à des opérations réalisées sur un ensemble de tels objets biologiques.

Par objets biologiques ou chimiques, il est entendu toute sorte d'objet moléculaire, supra moléculaire, cristallin, colloïdal, cellulaire, subcellulaire incluant de manière non exhaustive les cellules, les organelles, les virus, de l'ADN naturelle modifiée ou artificielle, de l'ARN ou d'autres acides nucléiques, des protéines, des glycoprotéines, des phosphoprotéines, des protéines artificielles ou naturelles, des lipides, des phospholipides, des molécules organiques, des molécules organométalliques, des macromolécules, des cristaux isolés, des quantum dots ou points quantiques, des nanoparticules, des vésicules, des microcapsules, et autres.

L'invention peut, en outre, être particulièrement utile en combinaison avec des méthodes optiques diverses, notamment des méthodes de détection optique.

Le procédé est applicable par exemple pour déterminer la présence d'un élément cible 37 dans une solution, la concentration d'un élément cible 37 dans une solution ce qui permet d'établir des caractéristiques d'affinités d'un élément cible 37 avec une autre espèce présente sur les particules 12 ou dans la goutte 6.

L'invention permet également la mesure de la constante de dissociation Kd entre l'élément cible 37 et une entité de signalisation 34.

Dans ce cas, la goutte 6 comprend en outre des entités de quantification qui présente une fluorescence différente de l'entité de signalisation 34.

Plus précisément, la définition de la constante de dissociation Kd du couple A :B entre l'élément A (élément cible 37) et l'élément B (entité de signalisation 34) est :

$$Kd=[A_{libre}][B_{libre}]/[A:B]$$

Où :

[$A_{libre}$] est la concentration de A non fixé à B en solution,
[$B_{libre}$] est la concentration de B non fixé à A en solution,
[A :B] est la concentration du complexe A :B en solution, dans lequel A est fixé à B.

La concentration totale de l'espèce A est :

$$[A_{total}]= [A_{libre}]+ [A:B]$$

La concentration totale de l'espèce B est :

$$[B_{total}]= [B_{libre}]+ [A:B]$$

La concentration [$B_{total}$] de l'entité de signalisation 34 dans la goutte 6 est choisie par l'utilisateur et connue.
La concentration [$A_{total}$] peut ne pas être connue a priori, par exemple lorsque A est un élément cible 37 sécrété par une cellule 90 encapsulée dans la goutte 6.
L'invention permet de mesurer la concentration du complexe [A :B] relocalisé sur l'agrégat 10 via le signal de l'entité de signalisation 34.
Cette mesure peut donner accès à une estimation de la constante de dissociation Kd entre A et B dans des conditions de concentrations maîtrisées, où l'on réalise la quantification de A via une entité de quantification qui est une autre entité de signalisation 34.
Un cas simple pour ce faire est de se placer dans le cas où :

- l'élément de capture 36 est en quantité suffisante et d'affinité suffisante pour l'élément cible 37 pour capturer plus de 90 % de l'élément cible 37, A sur l'agrégat 10, avantageusement la totalité,
- la concentration de l'entité de signalisation 34 est supérieure à celle de l'élément cible 37,
- la concentration de l'entité de quantification utilisée pour quantifier A est supérieure à celle de l'élément cible 37 et la constante de dissociation entre l'entité de quantification et l'élément cible 37 est inférieure à la concentration de l'élément cible 37, avantageusement d'un facteur supérieur à 10.

Dans ces conditions particulières, la présence de chaque élément cible 37 donne lieu à la formation d'un complexe élément de capture 36 - élément cible 37 - entité de quantification ou/et à la formation d'un complexe formé de l'élément de capture 36 - élément cible 37 - entité de signalisation 34.
Le nombre de complexe élément de capture 36 - élément cible 37 - entité de signalisation 34 est directement lié au Kd de l'élément cible 37 pour l'entité de signalisation 34 par l'équation suivante :

$$K_d = A * \frac{B}{[A:B]} = (A_{total} - [A:B]) * (B_{total} - [A:B])/[A:B]$$

Soit

$$[A:B] = (A_{total} + B_{total} + K_d - \sqrt{(A_{total} + B_{total} + K_d) - 4 * A_{total}B_{total}})/2$$

Il existe un régime dans lequel, $B_{total}$ étant fixé, la variabilité de $A_{total}$ est négligeable, et la seule mesure du rapport [A :B]/$A_{total}$ permet d'inférer Kd. Ainsi lorsque $A_{total} < B_{total}$, [A :B]/$A_{total}$ est une excellente approximation de

$$\frac{1}{1+K_d/B_{total}}.$$

La figure 10 représente des simulations du rapport [A :B] /$A_{total}$ en fonction du log Kd pour différentes valeurs de ratio $A_{total}$ /$B_{total}$.

Cet accès au Kd via le rapport [A :B]/$A_{total}$ représente un avantage clair pour une mesure haut-débit, car on peut évaluer le rapport [$A$:$B$]/$A_{total}$ par le ratio du signal de l'entité de signalisation 34 qui correspond au complexe A:B sur le signal de l'entité de quantification qui correspond à $A_{total}$ dans le cas particulier précité.

Ce ratio peut-être acquis en temps réel sur les gouttes et constituer un critère de tri des gouttes selon le Kd.

Le procédé est applicable pour déterminer la spécificité de l'élément cible 37 vis-à-vis de diverses entité de signalisation 34, ou la spécificité d'une molécule ou d'un assemblage de molécules fixé par l'élément de capture 36 vis-à-vis de diverses entité de signalisation 34. Dans ce cas, plusieurs entités de signalisation 34 par exemple des molécules marquées avec des fluorophores de couleurs différentes sont injectées dans la goutte 6. On pourra enregistrer en simultané le signal généré pour chaque entité de signalisation 34, par exemple en acquérant la fluorescence pour différentes longueur d'ondes. Il sera ainsi possible de déterminé quelles entités de signalisation 34 sont localisées sur l'agrégat 10 et de les quantifier.

Par exemple, l'élément de capture 36 est la protéine G, qui capture un anticorps 37 sécrété par une cellule 90, et les entités de signalisation 34 sont des antigènes homologues chez différentes espèces animale, chacun marqué par un fluorophore de couleur différente. Dans cet exemple, la mesure simultanée du signal pour chaque entité de signalisation 34 permet de savoir si l'élément cible 37 qu'est l'anticorps est spécifique d'une espèce animale (pic observé sur une seule couleur) ou non (pic observé sur plusieurs couleurs).

Dans un autre exemple, les particules 12 sont recouvertes d'une séquence de nucléotides propre à capturer une séquence de nucléotides complémentaires.

En variante, l'élément cible 37 est une protéine produite dans une goutte 6 ne comprenant pas de cellules mais un système de translation in vitro.

Le procédé est utile pour réaliser des analyses d'affinité et des quantifications de produits marqués. Par exemple, lorsque l'élément cible est une protéine fusion comprenant une protéine fluorescente comme la GFP.

Le procédé permet de plus le tri, la capture et l'extraction des gouttes présentant des caractéristiques intéressantes.

Dans un exemple, le procédé comprend la formation d'un sandwich; l'élément cible 37 étant d'une part lié à l'élément de capture 36 de la particule 14 et d'autre part à l'entité de signalisation 34, l'entité de signalisation 34 étant fluorescente.

Dans un exemple, l'élément de capture 36 est un anticorps.

Dans un exemple, l'entité de signalisation 34 est un anticorps.

Dans un exemple, l'élément cible 37 est une protéine.

En variante, l'élément cible 37 est un anticorps ou un fragment d'anticorps.

Dans un exemple particulier, l'élément cible 37 est un anticorps et l'élément de capture 36 est une protéine G, ou une protéine A ou une autre protéine fixant les anticorps, tel qu'une protéine A/G ou une protéine L.

Dans un exemple particulier, l'élément cible 37 est un anticorps et l'élément de capture 36 est un anticorps.

Dans un exemple, l'élément cible 37 est un anticorps et l'élément de capture 36 est un antigène reconnu par l'anticorps.

Dans un autre exemple, l'élément cible 37 est un anticorps et l'entité de signalisation 34 est un antigène.

Dans un autre exemple, l'élément de capture 36 est composée d'une pluralité de molécules de captures non identiques les unes aux autres et l'entité cible 37 est composée d'une pluralité de molécules non identiques les unes aux autres.

Dans un autre exemple, plusieurs paires élément de capture 36 - élément cible 37 ou triplets élément de capture 36 - élément cible 37 -entité de signalisation 34 peuvent être analysé simultanément, soit pour détecter la présence de plusieurs éléments cibles 37 de nature moléculaire différente, soit pour détecter et caractériser la présence de différents sites de fixation sur un même élément cible 37.

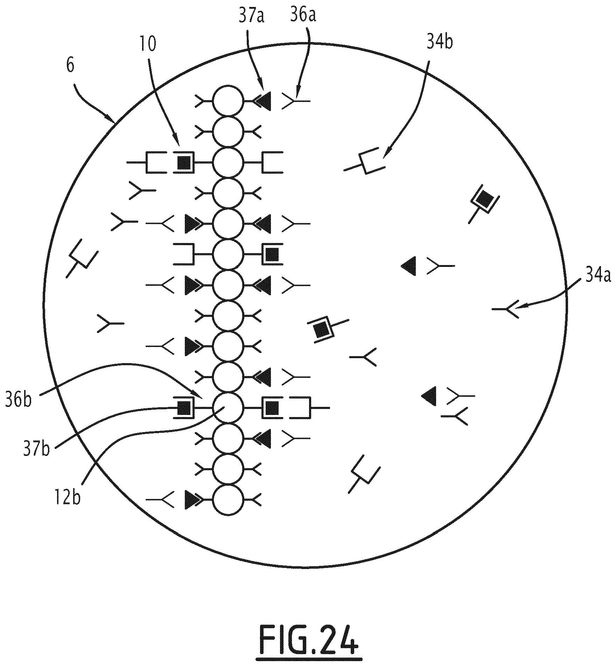

Par exemple, tel que représentée dans la goutte sur la figure 24, une goutte comprend plusieurs éléments cibles 37a, 37b de nature différente et plusieurs entités de signalisations 34a, 34b chacune étant apte à former un complexe avec un des éléments cibles 37a, 37b. Certaines particules 12 de l'agrégat 10 comprennent un élément de capture 36a adapté à la capture d'un premier élément cible 37a, d'autres particules comprennent un autre élément de capture 36b adapté à la capture d'un deuxième élément cible 37b.

Dans un exemple, le procédé permet de sélectionner selon les affinités mesurées des cellules immortalisées sécrétant des anticorps. Les cellules sélectionnées sont ensuite remises en culture.

Dans un autre exemple, le procédé permet de sélectionner selon les affinités mesurées des cellules non-immortalisées sécrétant des anticorps avant de rechercher les séquences de gènes des anticorps.

Dans un autre exemple, le procédé permet la quantification de cytokines sécrétées par différents types de cellules présentes dans le sang.

Dans certaines applications, l'agrégation des particules 12 est réversible.

Dans certains cas, la présence de l'élément cible 37 rend l'agrégation non réversible et consolide l'agrégat 10 lors de sa formation dans l'ensemble d'agrégation 30. L'agrégat 10 pré-existe donc de manière stable uniquement dans les gouttes 6 contenant l'élément cible 37. Dans les gouttes ne contenant pas l'élément cible 37, la réversibilité de l'agrégation des particules 12 dissous l'agrégat 10 tant qu'il ne rentre pas dans la zone de lecture 26. Dans un régime choisi d'aimantation et de fluidique, l'agrégat 10 ne peut se former et s'orienter qu'en présence de cette pré-agrégation, ce qui limite l'acquisition de pic selon la figure 2 aux gouttes contenant l'élément cible 37 par une autre méthode que via l'entité de signalisation 34.

Le procédé est notamment applicable pour cribler des cellules productrices d'anticorps en effectuant une mesure de l'affinité des anticorps produits. Par exemple si la cellule est une cellule B susceptible de produire des anticorps, l'affinité de cet anticorps pour au moins un antigène est déterminée par le procédé selon l'invention. Et les gouttes sont triées par le procédé selon l'invention selon, l'affinité déterminée.

Dans une autre application, le procédé permet l'analyse de la sécrétion d'une ou plusieurs cytokines par une population hétérogène de globules blancs. Par exemple si la cellule est une cellule susceptible de produire un ou plusieurs types de cytokines, les cytokines sont quantifiées par le procédé selon l'invention. Le résultat d'analyse constitue une information sur l'état de la cellule, avec une valeur diagnostique potentielle.

Des exemples de mise en oeuvre du procédé vont maintenant être décrits.

Le protocole des exemples suivants comprend :

- la préparation de plusieurs solutions aqueuses, contenant les particules 12, l'entité de signalisation 34 et l'élément cible ou avantageusement un système capable de le produire dans les gouttes l'injection des solutions aqueuses en entrée d'une puce de génération de gouttes,
- la génération de gouttes comprenant tous les réactifs de l'essai
- l'incubation de la solution contenant les gouttes,
- l'injection des gouttes dans un dispositif selon l'invention (deux types de dispositifs sont décrits ci-dessous dans les Exemple 1 et 2),
- la mesure des résultats de l'essai
- éventuellement un tri des gouttes en fonction de la mesure.

**Exemple 1 : dispositif de génération de gouttes et de mesure des gouttes de type 1**

La production des gouttes, autrement appelée compartimentation est réalisée après avoir mélangé une solution de réactifs et une solution d'échantillon sur puce.

Les solutions sont gardées sur la glace jusqu'à la compartimentation afin d'éviter toute dégradation des réactifs et échantillons.

La solution de réactifs est aspirée dans un réservoir connecté à une seringue Hamilton d'1 mL remplie d'huile minérale (Sigma Aldrich, #330760) juste avant le démarrage de la compartimentation.

Les échantillons à cribler sont mélangés à la solution de travail juste avant la compartimentation puis transférés dans une fiole en verre préalablement remplis d'huile fluorée (3M, NOVEC HFE-7500) et la fiole est maintenue à 4° sur glace.

Des capillaires, avantageusement en PTFE de diamètre interne 0.3mm (vendu par Fischer, #11919445) permettent de relier la fiole et le réservoir de la solution de réactifs au dispositif de formation de gouttes.

Ces deux solutions sont injectées sur une puce de formation de gouttes qui permet de générer des gouttes comprenant un volume égal de chacune des deux solutions.

Le volume des gouttes est choisi par l'utilisateur à partir du débit de l'huile fluorée. Avantageusement le volume des gouttes est de 33 picolitres. L'huile fluorée est le fluide porteur 8. Elle constitue la phase continue de l'émulsion comprenant les gouttes.

Les solutions de réactifs d'essais et d'échantillons à cribler sont injectées dans la puce au même débit, avantageusement à 200 microlitres/heure pour chaque solution. Le débit est imposé par un système standard de pompe de seringue par exemple une pompe neMESYS de Cetoni ou par une pompe contrôlant la pression par exemple le système commercialisé par Fluigent.

Les gouttes sont générées à une jonction de focalisation hydrodynamique telle qu'illustrée sur les figures 11 et 12. La phase externe est ici une huile fluorée (3M, NOVEC HFE-7500) à laquelle a été ajouté deux % poids/volume de tensioactifs (par exemple un copolymère tribloc comprenant deux queues perfluoropolyether (PFPE) (de poids moléculaire environ ~6,000 g/mol) et une tête PEG (~600 gmol)).

La figure 11 et la figure 12 représentent des dispositifs de flow-focusing permettant de mélanger un flux contenant les billes magnétiques mélangées aux autres réactifs et un flux contenant les échantillons avant la formation de gouttes au niveau de la jonction de focalisation hydrodynamique située à droite. Sur la figure 11, les particules magnétiques mesurent 500 nm de diamètre et sur la figure 12 les particules magnétiques mesurent 200 nm de diamètre.

Une deuxième étape est l'étape de collection. Une fiole maintenue à 4° sous le champ magnétique, avantageusement généré par un aimant en anneau (Amazing magnet H250H-DM), permet la collection des gouttes. Un capillaire court permet de connecter la fiole à la puce. Dans l'idéal, le capillaire de sortie mesure moins de 20 cm, avantageusement 10 cm.

Les gouttes sont mises à incuber avantageusement à 37°C pendant 20 à 90 minutes et sous champs magnétique, le temps et la température d'incubation dépendant de l'analyse effectuée et du type d'entité productrice 90 et d'élément cible 37 étudiée.

A la suite de l'incubation, la fiole contenant l'émulsion est transférée à 4° toujours maintenue sous champ magnétique.

Le premier type de dispositif, est un dispositif suivant l'invention tel que décrit sur la Figure 1.

La fiole contenant les gouttes est connectée à une puce pour une réinjection, la fiole est d'une part connectée à la puce et d'autre part à un système de pression, une pompe de pression ou une seringue et sa pompe, constituant l'ensemble de mise en circulation 22.

L'ensemble d'espacement 31 comprend deux entrées d'huiles connectées à la puce. Ces entrées sont destinées à injecter de l'huile avantageusement de l'huile fluorée permettant d'espacer les gouttes de l'émulsion telle qu'illustrée sur la figure 13.

Les débits de l'huile d'espacement sont avantageusement fixés chacun à 300 microlitres/heure ainsi que le débit de l'ensemble de mise en circulation est avantageusement fixé à 50 microlitres/heure et permettent d'ajuster le débit et la fréquence de réinjection de gouttes de façon à obtenir une fréquence comprise entre 250 et 1000 Hz.

Une paire d'aimants permanents 38 avantageusement fournie par K&J Magnetics, # BC 14-N52, est placée de part et d'autre de la puce autour du canal principal 24. Ces aimants 38 sont destinés à générer et à orienter les agrégats de billes pendant la réinjection des gouttes.

Un logiciel de contrôle de l'équipement, par exemple des lasers ou des photomultiplicateurs, est créé pour analyser et trier les gouttes. Le système de tri nécessite une carte FPGA pour réaliser une analyse en temps réel du signal.

La mesure est effectuée dans les gouttes une à une après leur passage dans l'ensemble d'espacement et ces gouttes peuvent être triées vers une sortie désirée après à zone de lecture illustré sur la figure 14.

Lorsqu'un tri et une récupération sont désirés, les gouttes triées et les émulsions non triées sont recueillies sur glace et le contenu des gouttes est récupéré à partir de protocoles standards.

**Exemple 2 : dispositif de mesure de gouttes de type 2.**

Le deuxième type de dispositif de mesure est une chambre de stockage des gouttes produite dans un plan à 2 dimensions. Cet exemple présente deux alternatives possibles pour réaliser de telles chambres.

La première est une chambre fabriquée par microfabrication classique en PDMS, comprenant avantageusement des piliers positionnés de manière régulière pour éviter l'effondrement de la chambre telle qu'illustrée sur les figures 3 à 6.

La seconde est une chambre en verre selon l'invention PCT/FR2009/051396 telle qu'illustrée sur les figures 15 et 16. Avantageusement, cette approche permet de réaliser une incubation des gouttes sur de longues durées (>1H) sans déformation des gouttes. Les gouttes peuvent donc être collectées directement dans une telle chambre après leur formation.

La figure 15 et la figure 16 illustrent un exemple de dispositif de lecture à deux dimensions selon l'invention, ici dans une chambre en verre, le champ magnétique est généré par un aimant permanent situé sur un côté de la chambre

**Généralités pour les Exemples suivants**

Dans les exemples suivants, la préparation des gouttes comprend :

- la préparation de deux solutions aqueuses, appelées « solution de réactifs » contenant les particules 12, l'entité de signalisation 34 et «solution d'échantillon à cribler » contenant l'élément cible dans les exemples ci-dessous,
- l'injection des deux solutions aqueuses en entrée de la puce de génération de gouttes,
- la génération de gouttes comprenant un volume égale de chacune des deux solutions,
- la mesure des gouttes dans un dispositif de type 1
- éventuellement le tri des gouttes (Exemple 4).

La préparation des deux solutions aqueuses va être décrite de manière générale et les différences par rapport à ce protocole standard seront mentionnées dans les Exemples le cas échéant.

a) Préparation de la Solution de réactifs

Les éléments compris dans cette solution sont avantageusement inertes les uns vis-à-vis des autres afin d'éviter les agrégations de réactifs avant la génération des gouttes.

Cette première solution aqueuse contient :

- des particules 12 qui sont ici des particules magnétiques colloïdales, fonctionnalisées avec un élément de capture 36, ici une protéine G, et
- un entité de signalisation 34 qui est ici un antigène marqué en fluorescence, par exemple un antigène marqué par le fluorophore Alexafluor488,
- un colorant permettant la détection des gouttes 6 par exemple la sulforhodamine B,
- un entité de quantification d'anticorps, qui est ici fluorescente, par exemple un fragment d'anticorps monoclonal, anti-souris marqué par le fluorophore Alexafluor647,
- une solution de travail.

Les concentrations de chaque réactif seront détaillées en regard de chaque exemple.

Ces réactifs sont dilués dans une solution appelée solution de travail. La solution de travail comprend par exemple:

- 30%v/v percoll (fourni par Sigma Aldrich),
- 50 mM de NaCl,
- 25 mM de tampon HEPES à pH 7,4,
- 0,1% v/v pluronic F-68 fourni par Life Technologies,
- 5%-v/v Serum Super low IgG de Thermo Scientific.

Le volume de la solution de travail est complété avec du RPMI-1640 complémenté de Glutamax ® fourni par Life Technologies pour atteindre le volume final.

Les particules 12 colloïdales magnétiques sont traitées avant usage. Les particules 12 sont des particules fournies par Chemicell (ScreenMAG) ou Ademtech (Bio Adembeads) dans une solution de stockage. Elles sont retenues sur un support magnétique afin de supprimer la solution de stockage puis elles sont mises en suspension dans un excès de pluronic F-127 à 10% poids/poids (Sigma Aldrich), avantageusement 10x le volume initiale de particules, et incubées pendant trente minutes à température ambiante.

Après ce traitement les particules 12 colloïdales magnétiques sont lavées deux fois dans du PBS et mises en suspension dans la solution de travail.

Avantageusement, les particules 12 en suspension dans la solution de travail sont soumises à une sonication pendant dix minutes avant l'ajout des réactifs d'essais.

Les réactifs fluorescents sont traités avant usage. Les réactifs fluorescents sont par exemple, l'élément de signalisation 34, le colorant de détection de goutte et le réactif de quantification. Les réactifs fluorescents sont centrifugés pendant cinq minutes à au moins 12.000 g et à 4°C afin de supprimer les traces d'agrégats de réactifs.

b) Préparation de la Solution d'échantillon à cribler

La solution d'échantillon à cribler comprend :

- un élément cible 37 propre à être capturée par l'élément de capture 36
- ou une entité productrice 90 pouvant synthétiser cet élément cible 37 dans la goutte pendant une phase d'incubation. Ce système peut être par exemple une cellule, ou un ADN et un système d'expression in vitro. Dans ce cas, l'élément cible 37 ne préexiste pas dans la solution d'échantillon à cribler.
- une solution de travail.

La concentration de cellules à utiliser dépend de la taille souhaitée pour les gouttes. Avantageusement, la concentration de cellules par goutte est de 0,3 cellules par goutte. Une émulsion avec des gouttes de 33 picolitres contient plus de $30.10^6$ gouttes par millitres. Pour des gouttes de 33 picolitres, pour avoir 0,3 cellules par goutte, et il faut environ $18.10^6$ cellules par millilitre de concentration dans la solution d'échantillon à cribler (qui est concentrée deux fois par rapport aux gouttes).

Il convient de noter que la concentration en cellule dans la solution d'échantillon à cribler est deux fois plus importante que la concentration finale puisque les deux solutions aqueuses seront mixées dans une goutte avec un ratio 50/50.

Le protocole de préparation des cellules dépend du type cellulaire et de l'objectif de l'expérience.

**Exemple 3 : Quantification et mesure d'affinité d'un anticorps monoclonal pour son antigène.**

Dans cet exemple, l'élément cible 37 est un anticorps qui est déjà contenu dans la solution à cribler, cet exemple ne met pas en oeuvre des cellules.

Un but de cet exemple est de démontrer la possibilité d'un test de liaison à plusieurs couleurs. Un autre but est de démontrer la possibilité de normaliser un signal de relocalisation avec un autre signal. Cela permet d'évaluer le $K_d$ à partir d'un signal de localisation d'un antigène (entité de signalisation) en permettant de normaliser par le signal de la protéine de liaison à chaîne légère de l'anticorps relocalisé (entité de quantification).

Dans cette expérience les gouttes mesurent 33 picolitres.

La solution d'échantillon à cribler contient différentes concentrations d'anticorps monoclonaux anti-hTNFα (fournis par Sigma Aldrich T6817) dilués dans du RPMI-1640 avec 2 mM de glutamax complémentés avec 30% v/v percoll, 0,1% v-v Pluronic F-68, 18 mM HEPES.

Les concentrations d'anticorps monoclonaux anti-hTNFα dans la solution d'échantillon à cribler sont les suivantes : 0 nM, 10 nM, 25 nM ou 50 nM.

La solution de réactifs contient les réactifs suivants :

- 8,33 % v/v de particules magnétiques (Ademtech #0433)
- 200 nM de Fab-DL650anti-Mouse Fab'2 (entité de quantification),
- 100 nM de hTNFα-AF488 (entité de signalisation),
- 1 μM de sulforhodamine B (pour le marquage des gouttes),

Le fragment d'anticorps Fab-DL650 est préparé à partir de Goat F(ab')2 anti-Mouse IgG (Fab')2 conjugated with DyLight-650 (commercialisé par Abcam, ab98760), digéré par la Papaïne et purifié sur une colonne de protéine G.

Cette solution est complétée de RPMI-1640 + 2mM Glutamax complémenté avec 30% v/v percoll, 0,1% v/v pluronic F-68 et 18 mM HEPES.

Cela permet d'obtenir quatre émulsions différentes avec 50 nM antigène-AF488, 100 nM de Fab-DL650 et respectivement 0 nM, 5 nM, 12,5 nM ou 25 nM d'anticorps monoclonaux dirigés contre l'antigène.

Les gouttes sont ensuite analysées au moyen d'un dispositif de type 1.

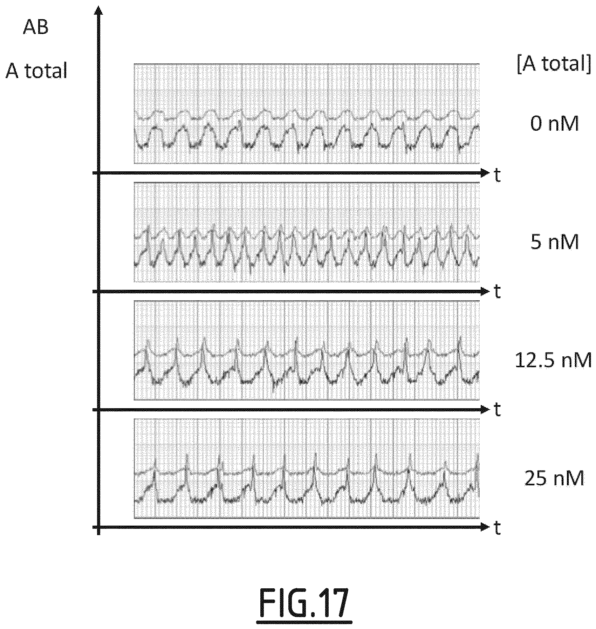

Cette expérience permet de montrer la possibilité de réaliser un test de liaison sur des particules magnétiques en colonne dans les gouttes avec deux lectures de fluorescence de réactifs secondaires, comme cela est illustré par la figure 17. De plus cette approche permet de caractériser l'affinité de liaison en corrélant le signal de liaison de l'antigène à la quantité d'anticorps impliqués dans la capture de l'antigène. En effet un paramètre indépendant de la concentration d'anticorps peut être extrait de l'analyse.

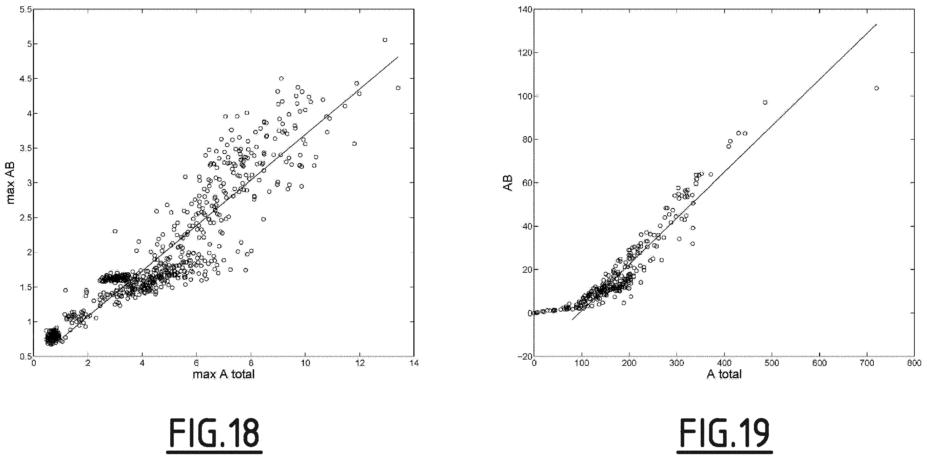

La figure 17 représente une mesure de fluorescence en temps réel sur un train de gouttes pour deux couleurs, une correspondant à l'entité de signalisation 34 (complexe AB, gris clair sur la figure 17) et une correspondant à l'entité de quantification, (composé A, gris foncé), et pour des quantités croissantes de A de 0 à 25 nM. La figure 18 est un graphique en deux dimensions correspondant à cette expérience, où chaque point est une goutte d'abscisse le maximum de fluorescence pour la couleur de l'entité de quantification, et en ordonnée le maximum de fluorescence pour l'entité de signalisation 34. La figure 19 représente pour le même jeu de gouttes un graphique en deux dimensions où chaque point a pour abscisse l'intégrale pour la couleur de l'entité de quantification, et en ordonnée l'intégrale pour l'entité de signalisation 34.

Nous constatons que la relation entre les signaux « maximum » est globalement linéaire malgré une dispersion des points de données (comme le suggère la régression linéaire avec $R^2=0.79$), due entre autres aux fluctuations de forme et de position de l'agrégat sur l'ensemble des gouttes. Nous constatons que la relation entre les signaux « intégrale » est caractérisée par une régression linéaire avec un meilleur $R^2$ (R2=0.90), la dispersion des points est nettement plus faible et permet d'estimer le Kd à partir du rapport AB/A comme précédemment expliqué.

**Exemple 4 : Tri d'hybridomes à l'échelle de la cellule unique en fonction de l'affinité des anticorps qu'ils sécrètent**

Le but de cette expérience est de démontrer le criblage de cellules productrices d'anticorps en fonction des affinités de liaison des anticorps monoclonaux secrétés en employant des réactifs et une démarche similaire à l'Exemple 3. En particulier, on montre qu'on peut différentier et trier deux hybridomes : un hybridome sécrétant un anticorps anti TNF alpha (lignée 25H12) et un hybridome sécrétant un anticorps anti c-Myc (lignée 9E10).

Dans cet exemple non seulement l'affinité est estimée mais en plus les cellules sont triées selon leur affinité.

Dans l'expérience les gouttes mesurent 33 picolitres.

La solution d'échantillon à cribler contient $13,5.10^6$ cellules d'hybridomes 9E10 et $4,5.10^6$ cellules d'hybridomes

25H12 mises en suspension dans la solution de travail décrite dans l'exemple 1.

La solution de réactifs contient les réactifs suivants mis en suspension dans la solution de travail :

- 8,33 % v-v magnetic particles (Ademtech #0433),
- 100 nM de fragments d'anticorps-DL650 anti-Mouse Fab'2 (entité de quantification),
- 100 nM de hTNF$\alpha$-AF 488 (entité de signalisation),
- 2 $\mu$M de sulforhodamine B.

Cela aboutit à une émulsion avec de 50 nM hTNFa-AF488, 50 nM de Fab-DL650 et des cellules d'hybridomes uniques sécrétant des anticorps dirigées soit contre l'hTNFa pour les hybridomes 25H12 soit contre le c-Myc pour les hybridomes 9E10. Les cellules 25H12 représentent 25% des cellules et les cellules 9E10 représentent 75% des cellules.

La fluorescence est mesurée pour les différents canaux de fluorescence, dans le vert pour l'antigène, et dans le rouge pour l'entité de quantification. Le signal considéré dans cet exemple est le maximum de fluorescence. Les gouttes présentant à la fois un signal de fluorescence vert et rouge important sont triées et recueillies.

Les gouttes triées et recueillies sont brisées et les cellules sont récupérées tel que décrit dans Mazutis et al. (Nat prot 2013).

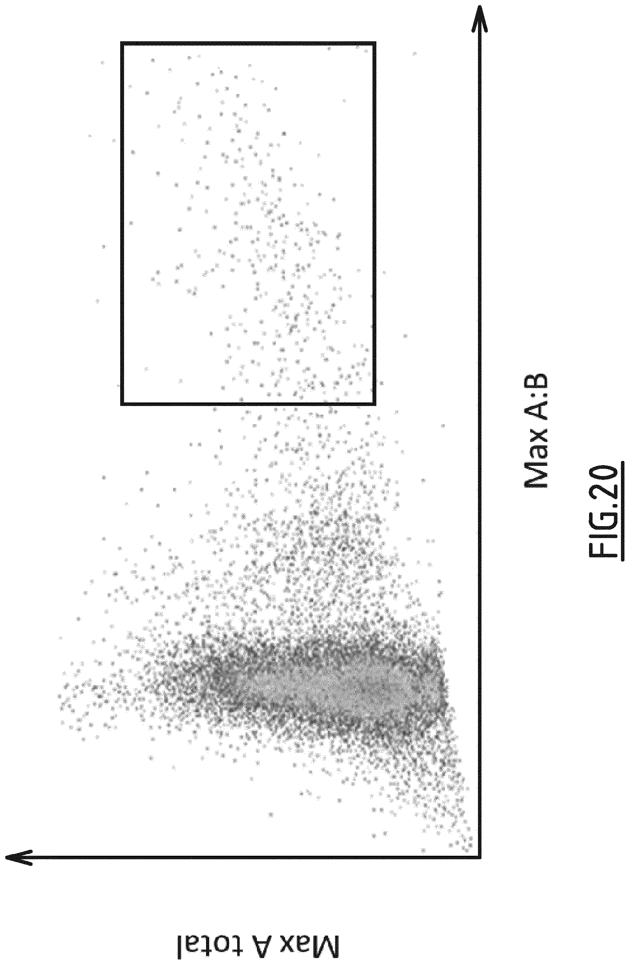

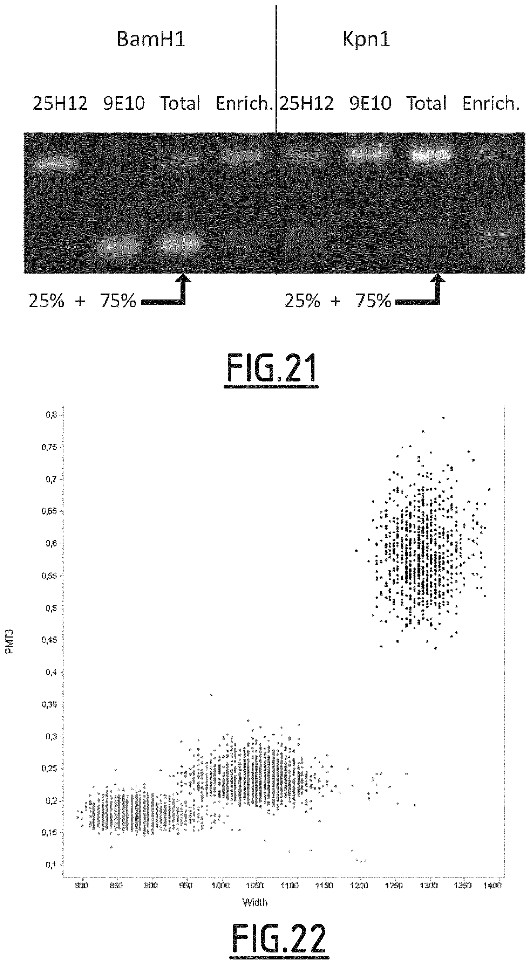

Une RT-PCR est ensuite réalisée sur les solutions de cellules extraites des gouttes triées ou de l'émulsion non triée, et les produits de PCR sont digérés en utilisant deux enzymes de restriction. L'enzyme BamHI a un site de restriction sur le cDNA provenant des cellules 9E10 et l'enzyme Kpnl a un site de restriction sur le cDNA provenant de 25H12. Les produits de digestions sont analysés par électrophorèse. Sur les figures 20 et 21 on voit que le procédé permet un enrichissement en cellules 25H 12 après le criblage par le dispositif.

L'invention permet donc une sélection spécifique à haut débit de cellules uniques sécrétant des anticorps reconnaissant l'entité de signalisation.

La figure 20 et la figure 21 illustrent un tri d'hybridomes selon l'invention. La figure 20 représente un graphique en deux dimensions où chaque point est une goutte d'abscisse le maximum de fluorescence pour la couleur de l'entité de signalisation, et en ordonnée le maximum de fluorescence pour l'entité de quantification. La fenêtre en noir indique la gamme de valeur sélectionnée pour effectuer le tri.

La figure 21 illustre une analyse par RT-PCR et digestion enzymatique par BamH1 (sur la série de quatre colonnes de gauche) ou Kpn1 (sur la série de quatre colonnes de droite) de l'ADN des hybridomes 25H12 (anti TNF alpha) et 9E10 (anti cMyc) purs (sur les deux premières colonnes de chaque série), ou dans un mélange 25/75 (sur la troisième colonne de chaque série) et enfin après enrichissement de ce mélange selon l'invention avec un bio-essai de reconnaissance du TNF alpha (sur la quatrième colonne de chaque série). Il y a un enrichissement d'un facteur d'environ 20 en cellules 25H12 après le tri.

## Exemple 5 : Quantification de cytokine secrétée à l'échelle de la cellule unique

Le but de cet exemple est de démontrer la possibilité de détecter et quantifier une cytokine sécrétée à l'échelle de la cellule unique avec une approche générique de tests immunologiques en sandwich. Dans cet exemple l'élément de capture 36 est un anticorps biotinylé fixé à la streptavidine et l'entité de signalisation 34 est un anticorps de détection fluorescent.

Les particules sont fonctionnalisées avec de la streptavidine et les anticorps de capture sont biotinylés. Il est possible d'utiliser une autre méthode de couplage de l'anticorps de capture sur les particules.

La méthode de couplage est covalente ou non covalente. Le critère important est que les entités de signalisation ne soient pas capturées directement à la surface des particules.

Il est présenté ici une méthode pour quantifier la sécrétion d'interféron gamma (IFNy), une cytokine biomarqueur dans de nombreux cas d'inflammation ou d'infection, à l'échelle de la cellule unique, selon l'invention et avec un dispositif de mesure de type 1. Un tel dosage biologique peut être utilisé pour tester le fonctionnement du système immunitaire de patients dans le cadre de tests d'immunologie fonctionnelle. Dans de tels tests les cellules immunitaires sont stimulées et leur sécrétion en cytokine est mesurée.

Dans cet exemple, nous avons démontré la possibilité de doser l'IFN$\gamma$ recombinant dans des gouttes mesurant 25 à 40 pL de volume.

Mais in fine, ce dosage a pour vocation d'être utilisé pour des cellules encapsulées individuellement, et le protocole général pour ce faire est maintenant décrit.

Chaque goutte contient tous les éléments nécessaires au système d'immuno-sandwich : le sandwich est par exemple composé de billes fluorescentes magnétiques recouvertes de streptavidine, d'anticorps biotinylés et d'anticorps fluorescents. Les deux sortes d'anticorps sont dirigées contre deux différents épitopes de l'IFN$\gamma$.

La solution de travail contient du milieu de culture et des activateurs de cellules (par exemple des mitogènes, des antigènes).

Une fois que les cellules et le système du sandwich d'immuno-essai sont encapsulés dans les gouttes, l'émulsion est incubée. Sous l'action du champ magnétique, les particules s'orientent au sein de la goutte et forment une colonne. Dans le cas d'un couplage non covalent de l'anticorps de capture par un système streptavidine-biotine, l'agrégation des billes en colonne est rendue irréversible par le pontage des billes via de multiples biotines situées sur l'anticorps de capture.

L'incubation permet la sécrétion et éventuellement la liaison des molécules entre elles. Après l'incubation, les gouttes sont analysées à l'aide du dispositif de type 1 selon l'invention.

Les gouttes comprenant des cellules ne répondant pas au signal d'activation et ne sécrétant pas d'interféron gamma, comportent des anticorps fluorescents au sein de toute la goutte. Les gouttes sécrétant des interférons gamma présentent des sandwiches anticorps de capture biotinylés - IFNγ - anticorps de détection fluorescent qui sont relocalisés sur la surface streptavidine des billes. On mesure donc dans ces gouttes une fluorescence relocalisée au niveau de l'agrégat.

Plus d'interférons gamma sont sécrétés, plus la fluorescence est sur la ligne de particules et plus le pic de fluorescence et/ou l'intégrale du signal de fluorescence sont élevés. Ceci permet donc une quantification des interférons gamma sécrétés par les cellules stimulées.

Dans cet exemple, nous avons montré qu'il est possible de réaliser un test de détection et quantification de l'interféron gamma selon l'invention en utilisant les réactifs suivants :

Solution de réactifs :

- Anticorps de capture biotinylé (Mabtech 7-B6-1) à 200 nM
- Anticorps de détection marqué par la phycoérythrine (PE) (Miltenyi 45-15) à 200 nM (entité de signalisation)
- Solution de travail : 30% percoll (Sigma) / 1% Pluronic F-68 (LifeTech) / 69% PBS (Sigma)

Solutions à cribler :

- 0 ou 20 ou 200 nM d'IFNγ 1B recombinant (Miltenyi)
- Billes magnétiques screenMAG/R Streptavidine 0.5μm (Chemicell) (20μl pour 120μl d'émulsion)

Un dispositif semblable à celui de l'Exemple 1 et 3 est utilisé pour générer les gouttes à partir des trois solutions à cribler, en jouant sur les flux pour obtenir une taille différente pour chaque concentration d'IFNγ (mais toujours en gardant les deux flux d'injection des solutions aqueuses égales pour obtenir la concentration finale souhaitée). Les gouttes font environ 25pL pour 0nM d'IFNγ, 31pL pour 10nM final, et 38pL pour 100nM final. Ces 3 populations de gouttes sont mélangées, incubées, et analysées pour mesurer la relocalisation de l'anticorps de détection orange. Le protocole identique est à celui de l'Exemple 3. La fluorescence est mesurée pour 2 canaux de fluorescence : orange pour l'anticorps de détection (PMT3), rouge pour les billes (PMT4). Le signal considéré dans cet exemple est le maximum de fluorescence.

Nous avons éliminé de l'analyse les gouttes pour lesquelles les valeurs sur le PMT4 (billes) sont faibles (PMT4<0.34), ce qui correspond au cas où la ligne de billes magnétiques est mal formée ou orientée.

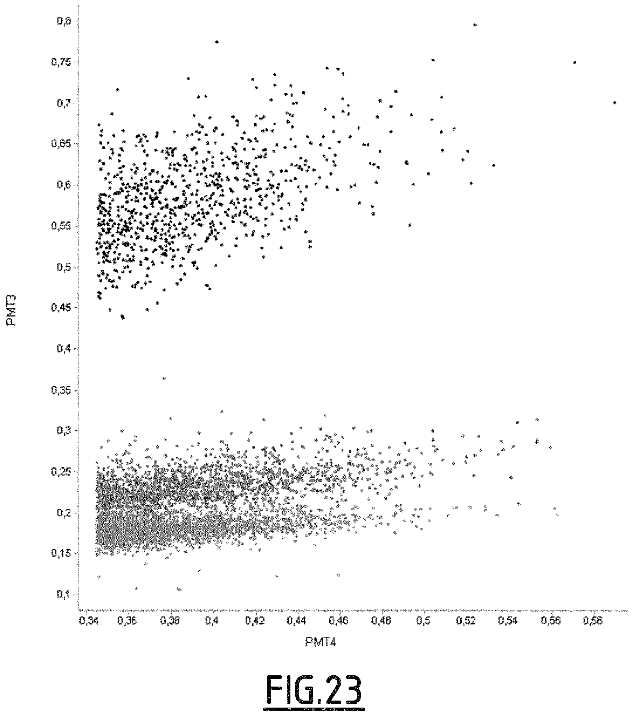

L'analyse des gouttes restantes montre qu'il est possible de discriminer les 3 populations de gouttes en taille et en signal d'anticorps de détection (PMT3), ce qui démontre la possibilité de quantifier l'IFNγ dans les gouttes comme illustré par la figure 22 et par la figure 23.

Elle montre aussi une relation linéaire entre le signal sur PMT3 et PMT4, ce qui suggère que la quantité d'anticorps de détection par bille magnétique est constante et que la variation du signal de l'entité de signalisation (PMT3) est corrélée directement à la variation de la forme de l'agrégat.

La figure 22 et la figure 23 illustrent une détection et quantification de cytokine (Interféron gamma, IFNγ) dans des gouttes microfluidiques selon l'invention. Sur la figure 22, les trois populations de gouttes contenant 0, 10, ou 100nM d'IFNγ sont représentées selon leur fluorescence maximale dans le orange (PMT3), qui correspond à l'anticorps de détection, par rapport à leur taille (Width). Les trois populations de gouttes sont représentées sur la figure 23 selon leur fluorescence maximale dans le orange (PMT3), par rapport à leur fluorescence maximale dans le rouge (PMT4), qui correspond aux billes.

**Exemple 6 : Tri de cellules primaires, plus précisément de lymphocytes B (cellules plasma), à l'échelle de la cellule unique en fonction de l'activité de liaison des anticorps qu'elles secrètent.**

Le but de cette expérience est de démontrer le criblage de cellules primaires productrices d'anticorps en fonction de l'activité de liaison des anticorps monoclonaux secrétés en employant des réactifs et une démarche similaire à l'Exemple 4. En particulier, on montre qu'on peut différentier et trier des lymphocytes B en fonction l'activité de liaison des anticorps monoclonaux qu'ils sécrètent.

Les lymphocytes B ayant été préalablement extraits de la rate d'une souris et purifiés suivant une procédure standard (Pan B kit II #130-104-443, Miltenyi Biotec). Dans cet exemple, l'élément de capture 36 est un antigène biotinylé, plus précisément « TTbiotin » la protéine *Tetanus Toxoid* préalablement fonctionnalisé avec une biotine grâce à une procédure de marquage d'un kit standard par exemple fourni par ThermoFisher. Dans cet exemple, seule l'activité de liaison de l'anticorps à l'antigène cible est observé mais en plus les cellules sont triées selon leur activité de liaison.

Dans l'expérience les gouttes mesurent 40 picolitres.

Dans cet exemple la solution de travail comprend :

- 5%-v/v Serum Super low IgG (#SH30898.03, Thermo Thermo Scientific,
- 25 mM de tampon HEPES à pH 7,4,
- 0,1% v/v pluronic F-68 fourni par Life Technologies,
- 1% v-v Antibiotic-Antimycotic (#15240, ThermoFisher)

Le volume de la solution de travail est complété avec du DMEM-F12 fourni par Life Technologies pour atteindre le volume final.

Deux émulsion sont produites et mise en commun après production et avant l'analyse et le criblage. L'émulsion majoritaire (~10 Million de gouttes) est constituée des cellules à cribler ainsi que des réactifs de détection. La seconde émulsion, dites de control négatif, comprenant ~1 Million de gouttes, n'est constitué que des réactifs de détection. Les deux émulsions sont différentiable grâce l'utilisation de deux concentrations différentes d'un fluorophore orange, la Sulforhodamine B.

La solution d'échantillon à cribler contient $6,6.10^6$ cellules primaires purifiées mises en suspension dans une solution de travail tel que décrite dans l'exemple 1.

La solution de réactifs contient les réactifs suivants mis en suspension dans la solution de travail :

- 33,33 % v-v de particules magnétique Streptavidine (Ademtech #0433),
- 200 nM de Rabbit Fab'2 anti-FCmouse AF647, JacksonIR #315-606-146 (entité de quantification),
- 100 nM de TTbiotin (élément de capture 36),
- 0,8 ou 1,6 ou 2,4 $\mu$M de Sulforhodamine B (Sigma Aldrich #S1402).

Cela aboutit à une émulsion avec de 50 nM d'antigen biotinylé (TTbiotin), 100 nM de Rab.Fab'2.antiFCmouse AF647 et des cellules primaires uniques sécrétant, ou non, des anticorps dirigés contre l'antigen (Tetanus Toxoid).

La fluorescence est mesurée dans le rouge pour l'entité de quantification. Le signal considéré dans cet exemple est le maximum de fluorescence. Les gouttes présentant un signal de fluorescence rouge important, ayant la bonne taille et provenant de l'émulsion des cellules primaires (faible fluorescence orange de la sulforhodamine B) sont triées, recueillies et brisées puis les cellules sont récupérées comme dans l'exemple 4.

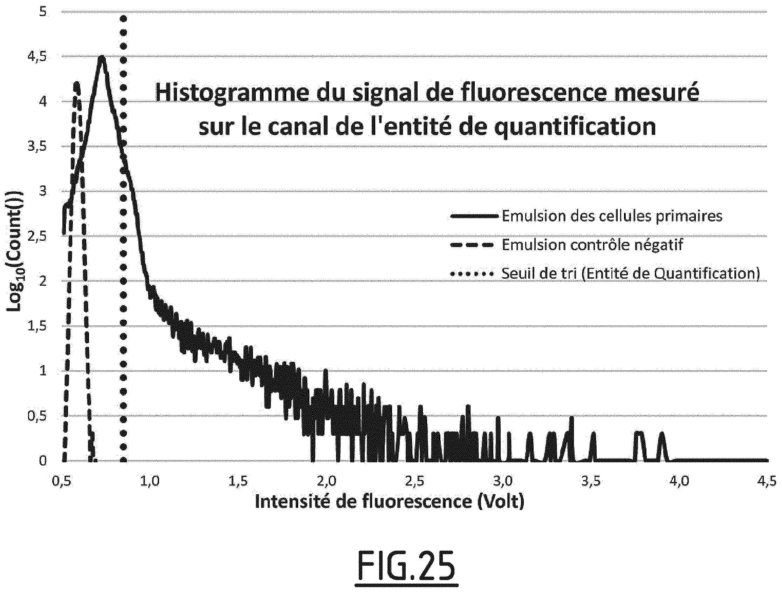

La figure 25 représente un histogramme du nombre de gouttes comptées pour un signal de fluorescence mesuré sur le canal de l'entité de quantification. L'abscisse représente le maximum de fluorescence pour la couleur de l'entité de quantification, et l'ordonnée représente le logarithme en base 10 du nombre de gouttes mesurée à cette valeur de fluorescence. Les valeurs obtenues pour l'émulsion de cellules primaires à cribler sont tracées en trait continu noir. Les valeurs obtenues pour l'émulsion de contrôle négatif sont tracées et trait pointillé gris. La ligne verticale de points ronds noirs indique la valeur seuil au-delà de laquelle les gouttes sont sélectionnées pour le tri.

Un ELISpot est ensuite réalisé sur les solutions de cellules extraites des gouttes triées ou de cellules non triées, c'est-à-dire après purification (kit Miltenyi) mais avant criblage microfluidique. Les cellules ainsi analysées par ELISpot sont testées à la fois pour la sécrétion d'anticorps et pour la sécrétion d'un anticorps anti-TT.

Une analyse par ELISpot de cellules primaires triées et de cellules primaires purifiés mais non-triées est réalisée. L'analyse est effectuée sur deux marqueurs en utilisant les procédures tel que décrites dans le kit Mouse IgG ELISpot[BASIC] (Mabtech #3825-2A). Le premier, dit IgG, utilise la procédure « Total Ig ELISpot » et permet de détecter le nombre de cellules sécrétant des anticorps. Le second, dit TT, utilise la procédure « Antigen-specific Ig ELISpot, PROTOCOL II » et permet de détecter le nombre de cellules sécrétant des anticorps ayant une activité de liaison contre l'antigène TT.

L'enrichissement $\eta$ est calculé selon la formule qui suit, $N_{+,0}$ étant le nombre de cellules positives avant tri, $N_{+,1}$ étant le nombre de cellules positives après tri, $N_{-,0}$ et $N_{-,1}$ étant les valeurs respectives de cellules négatives avant et après tri.

$$\eta = \frac{N_{+,1}}{N_{-,1}} \bigg/ \frac{N_{+,0}}{N_{-,0}}$$

Dans une expérience, on obtient pour 5000 cellules non triés 51 cellules positives à TT et 4949 cellules négatives et on obtient pour 1000 cellules triées, 132 cellules positives à TT et 868 cellules négatives.

L'expérience met en évidence un enrichissement, $\eta$, d'un facteur d'environ 15 en cellules sécrétrices ayant une activité de liaison à TT après le tri et ces cellules représentent 93% des cellules sécrétrices d'anticorps détectées.

Le test ELISpot permet de montrer que le procédé permet un enrichissement en cellules primaires après le criblage par le dispositif.

L'invention permet donc une sélection spécifique à haut débit de cellules primaires uniques sécrétant des anticorps reconnaissant l'élément de capture.

**Exemple 7: Quantification d'un anticorps monoclonal et détection de liaison avec son antigène en chambre 2D**

Dans cet exemple, l'élément cible 37 est un anticorps qui est déjà contenu dans la solution à cribler, cet exemple ne met pas en oeuvre des cellules.

Un but de cet exemple est de démontrer la possibilité de mesurer une réponse quantitative en fonction de la concentration d'un anticorps monoclonal pour un antigène donné dans un dispositif de type 60, c'est-à-dire une chambre dans laquelle les gouttes sont réparties en deux dimensions dans une couche unique. Dans cet exemple l'entité de signalisation est un antigène fluorescent, plus précisément « TT-AF488 » la protéine *Tetanus Toxoid* préalablement fonctionnalisé avec un fluorophore AlexaFluor-488 grâce à une procédure de marquage d'un kit standard par exemple fourni par ThermoFisher. Dans cet exemple les particules magnétiques utilisées pour former les colonnes en gouttes sont marqué à saturation avec l'entité de capture, ici le « CaptureSelectTM Biotin Anti-LC-kappa (Murine) conjugate » (ThermoFisher #7103152500).

Dans cette expérience les gouttes mesurent 40 picolitres.

Dans cet exemple la solution de travail comprend :

- 5%-v/v Serum Super low IgG (#SH30898.03, Thermo Thermo Scientific,
- 25 mM de tampon HEPES à pH 7,4,
- 0,1% v/v pluronic F-68 fourni par Life Technologies,

Le volume de la solution de travail est complété avec du DMEM-F12 dépourvu de rouge de phénol fourni par Life Technologies pour atteindre le volume final.

La solution de réactifs contient les réactifs suivants mis en suspension dans la solution de travail :

- 33,33 % v-v de particules magnétique Streptavidine (Ademtech #0433), préalablement marqué à saturation avec l'entité de capture
- 150 nM de Rabbit Fab'2 anti-FCmouse AF647, JacksonIR #315-606-146 (entité de quantification),
- 50 nM de TT-AF488 (entité de capture),
- 0,8 ou 1,6 ou 2,4 $\mu$M de Sulforhodamine B (Sigma Aldrich #S1402).

Cela aboutit à une émulsion avec de 25 nM d'antigène fluorescent (TT-AF488), 75 nM d'entité de quantification (Rab.Fab'2.antiFCmouse AF647) et une gamme de concentration d'un anticorps monoclonal, ayant une activité de liaison contre l'antigène (TT7), comprenant les valeurs suivantes : 0 ; 4,2 ; 12,5 ; 20,8 ; 42 (figure S7b seulement) ; 62,5 ; 83,3 ; 125 ; 208 ; 250 (figure S7b seulement). L'anticorps anti-TT, dit TT7, a été obtenu par expression de protéine recombinante à partir de la séquence publiée dans l'article écrit par Brandon J DeKosky et al., intitulé « High-throughput sequencing of the paired human immunoglobulin heavy and light chain repertoire » paru dans Nature Biotechnology Volume:31, pages 166 à 169 en 2013.

Les multiples émulsions mesurées sont différentiables grâce l'utilisation d'une gamme de concentrations d'un fluorophore orange, la Sulforhodamine B. Les mesures collectées n'ont pas été réalisées simultanément.

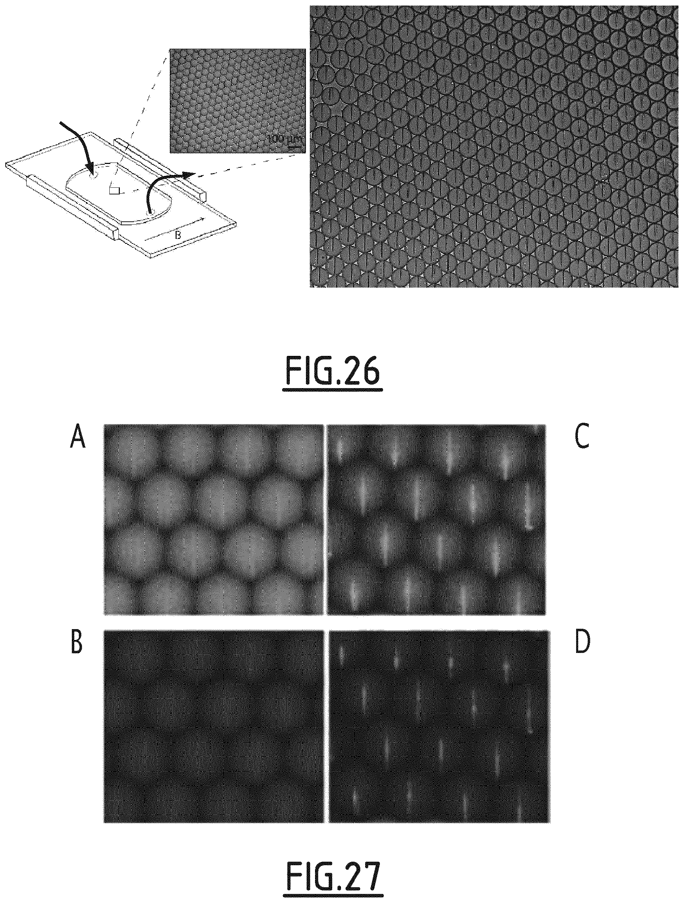

La figure 26 représente le dispositif de mesure du type du deuxième appareil 60 utilisé dans cet exemple. Une chambre de 40 $\mu$m de hauteur est créée entre deux lames de verre. Une entrée et une sortie sont réalisées dans la lame de verre supérieur et munis chacun de connecteur standard pour y connecter les capillaires de connexion.

Cette expérience permet de montrer la possibilité de réaliser un test de liaison sur des particules magnétiques en colonne dans les gouttes avec deux lectures de fluorescence de réactifs secondaires comme cela est illustré par la figure 27. En absence d'anticorps monoclonal on observe une fluorescence dispersée de l'entité de signalisation (figure 27 A) et de l'entité de quantification (figure 27 B). A l'inverse, en présence de 50nM d'un anticorps monoclonal ayant une activité de liaison contre l'antigène (TT7) on observe une relocalisation de fluorescence de l'entité de signalisation (Figure 27 C) et de l'entité de quantification (Figure 27 D).

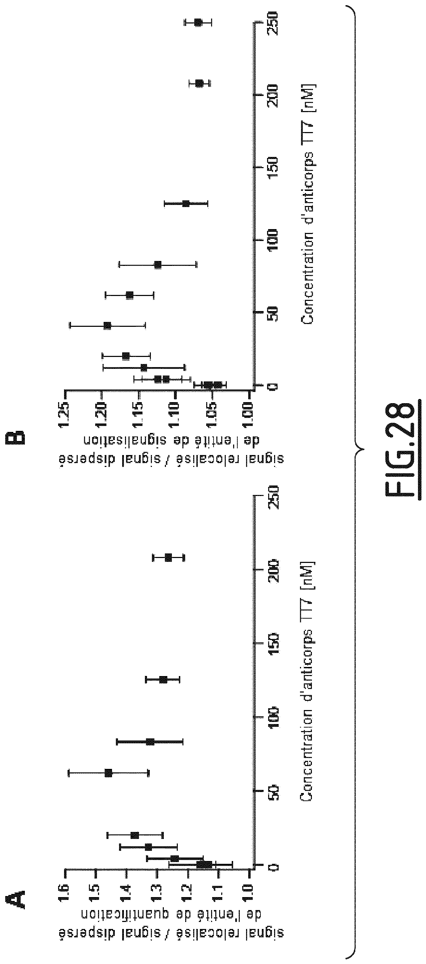

La figure 28 est une courbe de titration représentant le ratio des signaux relocalisé et dispersé des entités de quantification (figure 28 A) et de signalisation (figure 28 B) en fonction de la concentration d'élément cible (anticorps TT7) en nanomolaire.

**Exemple 8 : Mesure de l'affinité d'un anticorps monoclonal pour son antigène en chambre 2D**

Cet exemple est similaire en tout point à l'exemple 7 à la différence que plusieurs éléments cibles 37 distincts, représentant une gamme d'affinité pour l'antigène, sont mesurés.

Un but de cet exemple est de démontrer la possibilité de mesuré l'affinité d'un anticorps monoclonal pour un antigène donné dans un dispositif de type 60, c'est-à-dire une chambre dans laquelle les gouttes sont réparties en deux dimensions dans une couche unique.

La constante de dissociation Kd est évaluée à partir de la mesure de concentration d'antigène fluorescent lié à l'anticorps immobilisé sur la colonne de billes et de la mesure simultanée de la concentration d'anticorps capturée sur la colonne de billes.

Les anticorps anti-TT utilisé pour cette expérience, dit TT4, TT7 et TT10, ont été obtenus par expression de protéines recombinantes à partir des séquences publiées dans l'article écrit par Brandon J DeKosky et al., intitulé « High-throughput sequencing of the paired human immunoglobulin heavy and light chain repertoire » paru dans Nature Biotechnology Volume:31, pages 166 à 169 en 2013.

Différentes concentrations de chacun des trois anticorps ont été utilisées dans plusieurs émulsions distinctes afin d'obtenir par régression linéaire le ratio du signal de l'entité de signalisation sur le signal de l'entité de quantification respectivement pour chacun des trois anticorps, plus précisément 0 nM, 5 nM et 10 nM pour l'anticorps TT10, 0 nM, 2,5 nM et 10 nM pour l'anticorps TT4 et 0 nM, 5 nM, 10 nM, 15 nM et 25 nM pour l'anticorps TT7.

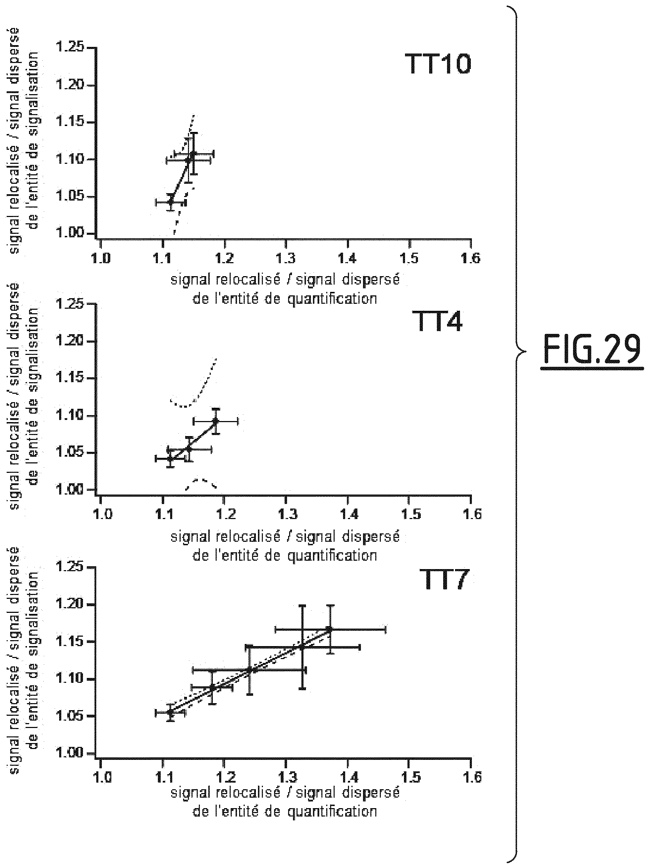

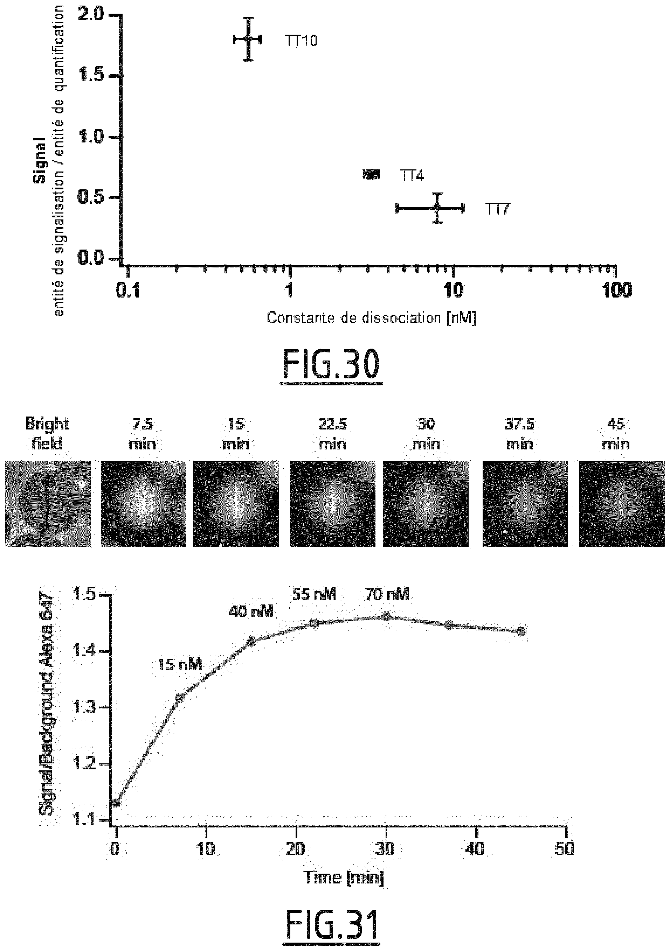

Cette expérience permet de montrer la possibilité de corréler le ratio du signal de l'entité de signalisation sur le signal de l'entité de quantification à la constante de dissociation de l'élément cible choisi comme cela est illustré par la figure 29 et 30.

La figure 29 est un graphique représentant le ratio des signaux relocalisé et dispersé des entités de quantification en abscisse et de signalisation en ordonnée pour les trois anticorps monoclonaux, TT4, TT7 et TT10 testés dans cette expérience.

La figure 30 est un graphique représentant en abscisse la constante de dissociation des trois anticorps monoclonaux obtenue par résonnance plasmonique de surface (SPR) et en ordonné leur ratio du signal de l'entité de signalisation sur le signal de l'entité de quantification.

**Exemple 9 : Mesure cinétique et quantitative de la sécrétion d'un anticorps monoclonal par des cellules primaires en chambre 2D**

Cet exemple est similaire en tout point à l'exemple 8 à la différence que l'élément cible 37, est un anticorps monoclonal sécrété par une cellule primaire et que cette sécrétion est mesurée de façon cinétique.

Un but de cet exemple est de démontrer la possibilité de mesuré simultanément la cinétique de sécrétion et l'affinité, pour un antigène donné, d'anticorps monoclonaux générés par des cellules primaires unique isolées en gouttes dans un dispositif de type 60.

Comme pour l'exemple 6, les lymphocytes B ont été préalablement extraits de la rate d'une souris et purifiés suivant une procédure standard (Pan B kit II #130-104-443, Miltenyi Biotec).

La figure 31 est un graphique représentant l'évolution, à l'échelle de la cellule unique, du ratio du signal relocalisé de la ligne de billes magnétiques sur le signal dispersé de la goutte en fonction du temps (min). De plus, grâce à la courbe de titration (Figure 28a de l'exemple 7), il est possible de corréler le signal mesuré à une concentration d'anticorps monoclonal. Cette concentration est notée sur la figure 30.

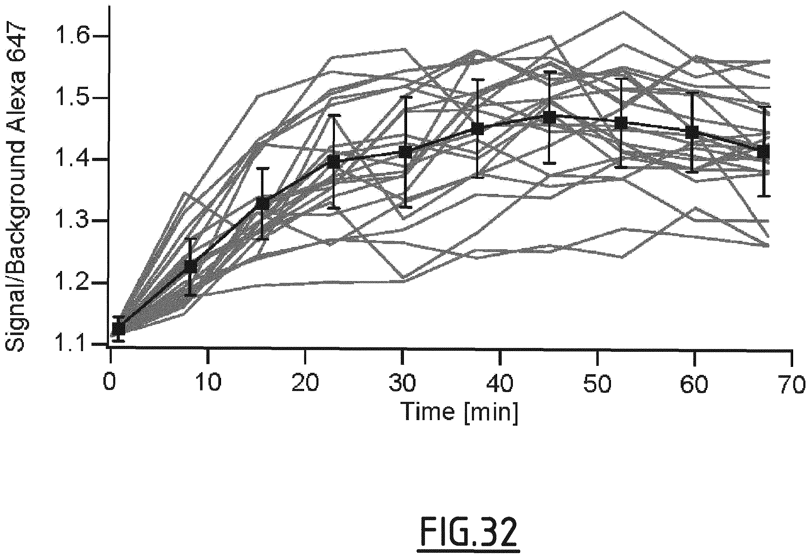

La figure 32 est un graphique représentant l'évolution, à l'échelle de la cellule unique, du ratio du signal relocalisé de la ligne de billes magnétiques sur le signal dispersé de la goutte en fonction du temps (min) pour n = 25 cellules primaires distinctes. La figure 32 illustre l'hétérogénéité des cellules primaires.

**Exemple 10 : Nombre, géométrie et stabilité des agrégats de billes magnétiques en goutte.**

Dans cet exemple, l'élément cible 37 est une molécule fluorescente biotinylé, par exemple AF546-Biotin (ThermoFisher #S11225) qui a aussi le rôle d'entité de signalisation et qui est déjà contenu dans la solution à cribler. Cet exemple ne met pas en oeuvre des cellules.

Un but de cet exemple est de démontrer la possibilité de stabiliser les lignes d'agrégats de billes magnétiques et d'assurer leurs singularités en goutte, grâce à l'utilisation d'une molécule de liaison transversale. Dans le cas de cette expérience nous montrons l'utilisation de différentes concentrations d'une protéine multiplement biotinylé, la Biotin-BSA (Sigma Aldrich #A8549) comme molécule de liaison transversale. La Biotin-BSA utilisé présente entre 8 et 16 mol de Biotine par mol d'Albumine.

Dans cette expérience les gouttes mesurent 40 picolitres.

Dans cet exemple la solution de travail comprend :

- 5%-v/v Serum Super low IgG (#SH30898.03, Thermo Thermo Scientific,
- 25 mM de tampon HEPES à pH 7,4,
- 0,1% v/v pluronic F-68 fourni par Life Technologies,
- 1% v-v Antibiotic-Antimycotic (#15240, ThermoFisher)

Le volume de la solution de travail est complété avec du DMEM-F12 fourni par Life Technologies pour atteindre le volume final.

Les sept émulsions produites pour cette expérience, contiennent les réactifs suivants mis en suspension dans la solution de travail :

- 100 nM final d'AF546-Biotin (entité de signalisation)
- 1 fois, 2 fois ou 3 fois la quantité standard de 33,33 % v-v de particules magnétique Streptavidine (Ademtech #0433),
- 50, 100, 200, 400, 800 ou 1600 nM de Dy-649 (Dyomics)
- Un ratio variable de Biotin-BSA, bille magnétique, de 0 pour 1, 5 pour 1, 25 pour 1 ou 100 pour 1, c'est-à-dire une concentration de Biotin-BSA de 0, 0.5, 2.5, 5 ou 15 nM.

Une émulsion de référence, dites « VHH lines », est produit comme décrit dans l'exemple « C ». Dans le cas de l'émulsion « VHH lines » la stabilisation des lignes de billes magnétique est obtenue grâce à la liaison transversale entre les éléments cible (anticorps) et les entités de capture (« CaptureSelectTM Biotin Anti-LC-kappa (Murine) conjugate » ThermoFisher #7103152500), parce qu'un même élément cible (anticorps) peut être lié à deux entités de capture.

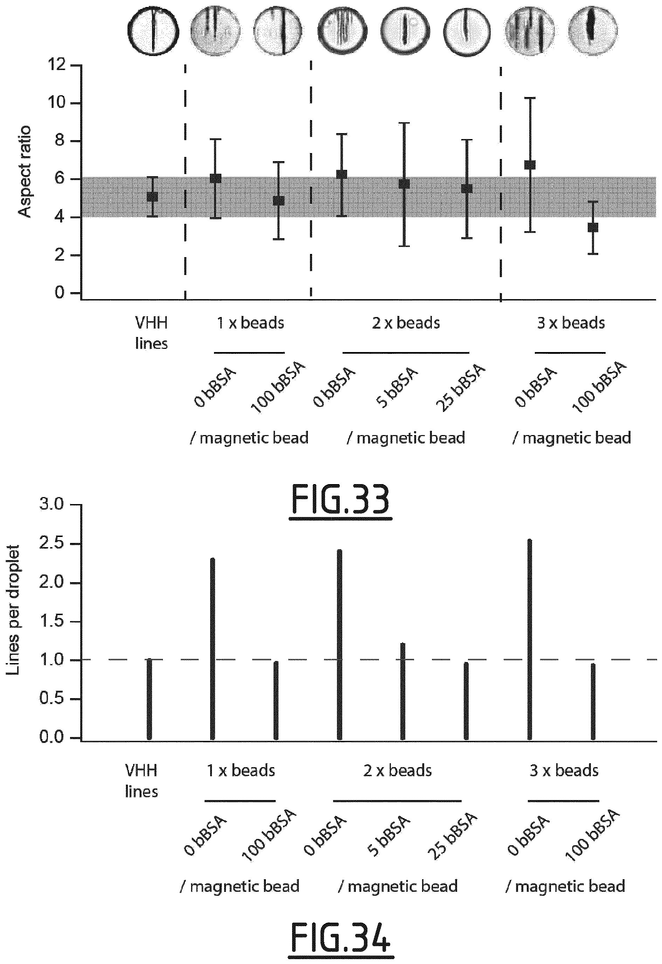

La figure 33 est un graphique représentant le rapport de format de la ligne de billes magnétiques en fonction des différentes conditions expérimentales et illustré dans chaque cas par une image de microscopie.

La figure 34 est un graphique représentant la singularité ou pluralité de la ligne de billes magnétiques en fonction des différentes conditions expérimentales.

Cette expérience permet de montrer la possibilité de stabiliser le nombre et la géométrie des lignes de billes magnétiques par goutte comme cela est illustré par les figures 33 et 34.

## Revendications

1. Procédé d'analyse du contenu de gouttes, comprenant l'étape suivante :

   - fourniture d'une pluralité de gouttes (6) contenues dans un fluide porteur (8), au moins une des gouttes (6) comprenant au moins un agrégat (10) de particules (12) définissant un objet allongé selon un axe principal (X), au moins certaines gouttes (6) contenant au moins un élément cible (37) propre à se fixer sur l'agrégat (10),

   **caractérisé en ce que** l'agrégat (10) de particules (12) comporte une colonne orientée selon l'axe principal (X) et **en ce que** le procédé comprend une étape de mesure d'un paramètre physique caractéristique de la fixation de l'élément cible (37) sur l'agrégat (10), l'étape de mesure comportant la mesure du paramètre physique localement en une pluralité de points situés dans la goutte (6), l'étape de mesure comportant de préférence la détermination de l'intégrale des valeurs mesurées au sein de la goutte (6).

2. Procédé selon la revendication 1, dans lequel les particules (12) sont des particules superparamagnétiques.

3. Procédé selon l'une des revendications 1 ou 2, dans lequel l'étape de fourniture des gouttes (6) comprend :

   - la dispersion des particules (12) dans une masse de fluide (86) destinée à former les gouttes (6), puis
   - la dispersion de la masse de fluide (86) sous forme de gouttes (32),

- la formation dans chaque goutte (6) d'au moins un agrégat (10) de particules (12) définissant un objet allongé selon un axe principal, l'agrégat (10) de particules (12) étant formé dans chaque goutte (6) après la dispersion.

4. Procédé selon l'une quelconque des revendications précédentes, dans lequel l'élément cible (37) est un élément choisi dans le groupe constitué par une protéine, un anticorps, un peptide, un morceau d'ADN ou d'ARN, un métabolite, un ion, un lipide et une biomolécule susceptible d'être produite par une cellule.

5. Procédé selon l'une quelconque des revendications précédentes, dans lequel au moins certains gouttes (6) comprennent une entité productrice (90) susceptible de produire l'élément cible (37), l'entité productrice (90) étant de préférence choisie dans le groupe constitué par une cellule et un système d'expression in vitro.

6. Procédé selon l'une quelconque des revendications précédentes, comprenant avant l'étape de mesure, une étape d'orientation de l'axe principal (X) de l'agrégat selon un axe de détection (D).

7. Procédé selon l'une quelconque des revendications précédentes, comprenant de multiples étapes de mesure, avec une étape d'orientation de l'axe principal (X) de l'agrégat selon un axe de détection différent pour chacune des mesures.

8. Procédé selon l'une quelconque des revendications précédentes, dans lequel l'étape de mesure est effectuée dans une chambre microfluidique sans circulation des gouttes.

9. Procédé selon l'une quelconque des revendications précédentes, comprenant :

   - la fourniture d'un dispositif comprenant un ensemble de mise en circulation (22) de la goutte et une zone de détection (26),
   - le transport de la goutte vers la zone de détection (26), la mesure au sein de la goutte (6) étant effectuée dans la zone de détection (26).

10. Procédé selon l'une quelconque des revendications précédentes, comprenant :

    - la fourniture d'un dispositif comprenant un ensemble de mise en circulation (22) de la goutte et une pluralité de zones de classement (94, 96), et un moyen de direction (98) de la goutte ou d'une partie de la goutte sélectivement vers une zone de classement (94, 96),
    - la décision de classement de la goutte (6) ou d'une partie de la goutte, la décision consistant à choisir sélectivement une zone de classement parmi la pluralité des zones de classement (94, 96),
    - le transport de la goutte (6), respectivement d'une partie de la goutte, vers la zone de classement de la goutte (6) choisie lors de l'étape de décision.

11. Procédé selon l'une quelconque des revendications précédentes, dans lequel au moins une goutte comprend au moins un élément cible (37), au moins une première entité de signalisation (34) apte à former un complexe avec l'élément cible (37) et au moins une seconde entité de signalisation (34) distincte apte à former un complexe avec l'élément cible (37), le procédé comprenant la mesure d'un signal indiquant la concentration de chacune des entités de signalisation (34) relocalisées sur l'agrégat (10).

12. Procédé selon l'une quelconque des revendications précédentes, dans lequel au moins une goutte comprend au moins un élément cible (37), au moins une entité de signalisation (34) apte à former un complexe avec l'élément cible (37) et au moins une entité de quantification apte à former un complexe avec l'élément cible (37), le procédé comprenant :

    - la mesure d'un signal représentatif de la concentration de l'entité de signalisation (34) relocalisée sur l'agrégat (10),
    - la mesure d'un signal représentatif de la concentration de l'entité de quantification relocalisée sur l'agrégat (10),
    - la détermination de la constante de dissociation de l'élément cible (37) avec l'entité de signalisation (34) à partir du rapport du signal de l'entité de signalisation (34) relocalisée sur le signal de l'entité de quantification relocalisée.

13. Procédé selon la revendication 12, dans lequel au moins certaines gouttes (6) comprennent une entité productrice (90), l'entité productrice (90) étant une cellule susceptible de produire au moins un anticorps étant un élément cible (37), le procédé comprenant une étape de détermination de l'affinité de l'anticorps produit par l'entité productrice (90) pour au moins un antigène, le procédé comprenant de préférence une étape de tri de la goutte après l'étape de détermination.

**14.** Procédé selon l'une quelconque des revendications précédentes, dans lequel au moins une goutte comprend au moins deux entités de signalisation (34) distinctes, chacune des deux entités de signalisation (34) étant apte à former un complexe avec un élément cible distinct (37) sur l'agrégat (10), le procédé comprenant la mesure d'un signal indiquant la concentration de chacune des entités de signalisation (34) relocalisée.

**15.** Procédé selon la revendication 14, dans lequel au moins certaines gouttes (6) comprennent une entité productrice (90), l'entité productrice (90) étant une cellule susceptible de produire un ou plusieurs types de protéines, chaque protéine étant un élément cible distinct (37), la mesure du signal indiquant la concentration de chacune des entités de signalisation (34) relocalisée permettant une quantification du ou des types de protéines.

**16.** Appareil d'analyse du contenu de gouttes comprenant :

- un ensemble de fourniture d'une pluralité de gouttes (6) contenues dans un fluide porteur (8), au moins une des gouttes (6) comprenant au moins un agrégat (10) de particules (12) définissant un objet allongé selon un axe principal (X),

**caractérisé en ce que** l'agrégat (10) de particules (12) comporte une colonne orientée selon l'axe principal (X) et **en ce que** l'appareil comprend un ensemble de mesure d'un paramètre physique caractéristique de la fixation d'un élément cible (37) sur l'agrégat (10), l'ensemble de mesure étant propre à mesurer le paramètre physique localement en une pluralité de points situés dans la goutte (6) et, de préférence, à déterminer l'intégrale des valeurs mesurées au sein de la goutte (6), l'appareil comprenant, en outre, de préférence :

- un ensemble mise en circulation de la goutte (22),
- un ensemble de décision de classement de la goutte,
- un ensemble de tri de la goutte selon la décision de classement.

**Patentansprüche**

**1.** Verfahren zur Analyse des Inhalts von Tropfen, folgenden Schritt umfassend:

- Liefern einer Mehrzahl von Tropfen (6), die in einem Trägerfluid (8) enthalten sind, wobei mindestens einer der Tropfen (6) mindestens ein Aggregat (10) von Partikeln (12) umfasst, das ein gemäß einer Hauptachse (X) längliches Objekt definiert, wobei mindestens einige Tropfen (6) mindestens ein Zielelement (37) enthalten, das geeignet ist, sich an dem Aggregat (10) zu fixieren,

**dadurch gekennzeichnet, dass** das Aggregat (10) von Partikeln (12) eine gemäß der Hauptachse (X) orientierte Säule aufweist und dass das Verfahren einen Schritt des Messens eines physikalischen Parameters, der für die Festlegung des Zielelements (37) an dem Aggregat (10) charakteristisch ist, wobei der Messschritt die Messung des physikalischen Parameters lokal an einer Mehrzahl von Punkten, die in dem Tropfen (6) liegen, aufweist, wobei der Messschritt vorzugsweise die Bestimmung des Integrals der an dem Tropfen (6) gemessenen Werte aufweist.

**2.** Verfahren nach Anspruch 1, bei dem die Partikel (12) superparamagnetische Partikel sind.

**3.** Verfahren nach einem der Ansprüche 1 oder 2, bei dem der Schritt des Lieferns der Tropfen (6) umfasst:

- Dispergieren der Partikel (12) in einer Fluidmasse (86), die zur Bildung der Tropfen (6) dient, dann
- Dispergieren der Fluidmasse (86) in Form von Tropfen (32),
- Bilden in jedem Tropfen (6) mindestens eines Aggregats (10) von Partikeln (12), das ein gemäß einer Hauptachse langgestrecktes Objekt definiert, wobei das Aggregat (10) von Partikeln (12) in jedem Tropfen (6) nach dem Dispergieren gebildet wird.

**4.** Verfahren nach einem beliebigen der vorhergehenden Ansprüche, bei dem das Zielelement (37) ein Element ist, ausgewählt aus der Gruppe, die aus einem Protein, einem Antikörper, einem Peptid, einem Stück ADN oder ARN, einem Metabolit, einem Ion, einem Lipid und einem Biomolekül, das geeignet ist, durch eine Zelle produziert zu werden.

5.  Verfahren nach einem beliebigen der vorhergehenden Ansprüche, bei dem mindestens einige Tropfen (6) eine produzierende Entität (90) umfassen, die geeignet ist, das Zielelement (37) zu erzeugen, wobei die produzierende Entität (90) vorzugsweise ausgewählt ist aus der Gruppe, die durch eine Zelle und einem Expressionssystem in vitro gebildet ist.

6.  Verfahren nach einem beliebigen der vorhergehenden Ansprüche, das vor dem Schritt des Messens einen Schritt des Orientierens der Hauptachse (X) des Aggregats gemäß einer Detektionsachse (D) umfasst.

7.  Verfahren nach einem beliebigen der vorhergehenden Ansprüche, das mehrere Messschritte mit einem Schritt des Orientierens der Hauptachse (X) des Aggregats gemäß einer Detektionsachse, die für jede der Messungen unterschiedlich ist, umfasst.

8.  Verfahren nach einem beliebigen der vorhergehenden Ansprüche, bei dem der Messschritt in einer mikrofluiden Kammer ohne Zirkulation von Tropfen durchgeführt wird.

9.  Verfahren nach einem beliebigen der vorhergehenden Ansprüche, umfassend:

    - Liefern einer Vorrichtung, die eine Anordnung (22) zum Zirkulieren des Tropfens und eine Detektionszone (26) umfasst,
    - Transportieren des Tropfens zu der Detektionszone (26), wobei die Messung in dem Tropfen (6) in der Detektionszone (26) durchgeführt wird.

10. Verfahren nach einem beliebigen der vorhergehenden Ansprüche, umfassend:

    - Liefern einer Vorrichtung, die eine Anordnung (22) zum Zirkulieren des Tropfens und eine Mehrzahl von Klassifizierungszonen (94, 96), und ein Mittel (98) zum Richten des Tropfens oder eines Teils des Tropfens selektiv zu einer Klassifizierungszone (94, 96) umfasst,
    - Entscheiden der Klassifizierung des Tropfens (6) oder eines Teils des Tropfens, wobei die Entscheidung darin besteht, selektiv eine Klassifizierungszone aus der Mehrzahl von Klassifizierungszonen (94, 96) auszuwählen,
    - Transportieren des Tropfens (6) respektive eines Teils des Tropfens zu der Klassifizierungszone des Tropfens (6), die bei dem Entscheidungsschritt ausgewählt ist.

11. Verfahren nach einem beliebigen der vorhergehenden Ansprüche, bei dem mindestens ein Tropfen mindestens ein Zielelement (37), mindestens eine erste Signalisierungsentität (34), die geeignet ist, einen Komplex mit dem Zielelement (37) zu bilden, und mindestens eine zweite unterschiedliche Signalisierungsentität (34), die geeignet ist, einen Komplex mit dem Zielelement (37) zu bilden, umfasst, wobei das Verfahren die Messung eines Signals umfasst, die die Konzentration jeder Signalisierungsentität (34), die sich auf dem Aggregat (10) positioniert hat, umfasst.

12. Verfahren nach einem beliebigen der vorhergehenden Ansprüche, bei dem mindestens ein Tropfen mindestens ein Zielelement (37), mindestens eine Signalisierungsentität (34), die geeignet ist, einen Komplex mit dem Zielelement (37) zu bilden, und mindestens eine Quantifizierungsentität, die geeignet ist einen Komplex mit dem Zielelement (37) zu bilden, umfasst, wobei das Verfahren umfasst:

    - Messen mindestens eines Signals, das für die Konzentration der Signalisierungsentität (34), die auf dem Aggregat (10) positioniert ist, repräsentativ ist,
    - Messen eines Signals, das für die Konzentration der Quantifizierungsentität, die auf dem Aggregat (10) positioniert ist, repräsentativ ist,
    - Bestimmen der Dissoziationskonstante des Zielelements (37) mit der Signalisierungsentität (34) aus dem Verhältnis des Signals der positionierten Signalisierungsentität (34) zu dem Signal der positionierten Quantifizierungsentität.

13. Verfahren nach Anspruch 12, bei dem mindestens einige Tropfen (6) eine produzierende Entität (90) umfassen, wobei die produzierende Entität (90) eine Zelle ist, die geeignet ist, mindestens einen Antikörper, der ein Zielelement (37) ist, zu erzeugen, wobei das Verfahren einen Schritt des Bestimmens der Affinität des von der produzierenden Entität (90) erzeugten Antikörpers für mindestens ein Antigen umfasst, wobei das Verfahren vorzugsweise einen Schritt des Sortierens des Tropfens nach dem Schritt des Bestimmens umfasst.

14. Verfahren nach einem beliebigen der vorhergehenden Ansprüche, bei dem mindestens ein Tropfen mindestens zwei unterschiedliche Signalisierungsentitäten (34) umfasst, wobei jede der zwei Signalisierungsentitäten (34) geeignet ist, einen Komplex mit einem unterschiedlichen Zielelement (37) auf dem Aggregat (10) zu bilden, wobei das Verfahren die Messung eines Signals umfasst, das die Konzentration jede der positionierten Signalisierungsentitäten (34) angibt.

15. Verfahren nach Anspruch 14, bei dem mindestens einige Tropfen (6) eine produzierende Entität (90) umfassen, wobei die produzierende Entität eine Zelle ist, die geeignet ist, eine oder mehrere Arten von Proteinen zu erzeugen, wobei jedes Protein ein unterschiedliches Zielelement (37) ist und die Messung des Signals die Konzentration jedes der gebundenen Signalisierungsentitäten (34) angibt, die eine Quantifizierung des oder der Arten von Proteinen gestattet.

16. Vorrichtung zur Analyse des Inhalts von Tropfen, umfassend:

- eine Anordnung zum Liefern einer Mehrzahl von Tropfen (6), die in einer Trägerflüssigkeit (8) enthalten sind, wobei mindestens einer der Tropfen (6) mindestens ein Aggregat (10) von Partikeln (12) umfasst, das ein gemäß einer Hauptachse (X) langgestrecktes Objekt definiert,

**dadurch gekennzeichnet, dass** das Aggregat (10) von Partikeln (12) eine Säule, die gemäß der Hauptachse (X) orientiert ist, aufweist, und dass
die Vorrichtung eine Anordnung zum Messen eines physikalischen Parameters, der für die Festlegung eines Zielelements (37) an dem Aggregat (10) charakteristisch ist, umfasst, wobei die Messanordnung geeignet ist, den physikalischen Parameter lokal an einer Mehrzahl von Punkten, die in dem Tropfen (6) liegen, zu messen, und vorzugsweise das Integral der in dem Tropfen (6) gemessenen Werte zu bestimmen, wobei die Vorrichtung außerdem vorzugsweise umfasst:

- eine Anordnung zum Zirkulieren des Tropfens (22),
- eine Anordnung zum Entscheiden über die Klassifizierung des Tropfens,
- eine Anordnung zum Sortieren des Tropfens gemäß der Entscheidung der Klassifizierung.

**Claims**

1. A method for analyzing the content of drops, comprising the following step:

- providing a plurality of drops (6) contained in a carrier fluid (8), at least one of the drops (6) comprising at least an aggregate (10) of particles (12) defining an elongated object along a main axis (X), at least certain drops (6) containing at least one target element (37) able to bind to the aggregate (10),

**characterized in that** the aggregate (10) of particles (12) comprises a column oriented along the main axis (X) and **in that** the method comprises a measurement step for measuring a physical parameter characteristic of the attachment of the target element (37) on the aggregate (10), the measurement step including the measurement of a physical parameter locally in a plurality of points located in the drop (6), the measurement step preferably including the determination of the integral of the measured values within the drop (6).

2. The method according to claim 1, wherein the particles (12) are superparamagnetic particles.

3. The method according to one of claims 1 or 2, wherein the step for providing the drops (6) comprises:

- the dispersion of the particles (12) in a fluid mass (86) intended to form the drops (6), and then
- the dispersion of the fluid mass (86) in the form of drops (32),
- the formation in each drop of at least one aggregate of particles defining an elongated object along a main axis, the aggregate (10) of particles (12) being formed in each drop (6) after the dispersion.

4. The method according to any of the preceding claims, wherein the target element (37) is an element selected from the group formed by a protein, an antibody, a peptide, a piece of DNA or RNA, a metabolite, an ion, a lipid and a biomolecule which may be produced by a cell.

5. The method according to any of the preceding claims, wherein at least certain drops (6) comprise a productive entity (90) which may produce the target element (37), the productive entity (90) being preferably selected from the group formed by a cell and an *in vitro* expression system.

6. The method according to any of the preceding claims, comprising before the measurement step, a step for orienting the main axis (X) of the aggregate along a detection axis (D).

7. The method according to any of the preceding claims, comprising multiple measurement steps, with a step for orienting the main axis (X) of the aggregate along a different detection axis for each of the measurements.

8. The method according to any of the preceding claims, wherein the measurement step is carried out in a microfluidic chamber without circulation of the drops.

9. The method according to any of the preceding claims, comprising:

   - providing a device comprising an assembly for putting in circulation (22) the drop and a detection area (26),
   - the transport of the drop towards the detection area (26), the measurement within the drop (6) being carried out in the detection area (26).

10. The method according to any of the preceding claims, comprising:

    - the provision of a device comprising an assembly (22) for putting in circulation the drop and a plurality of classification areas (94, 96), and a means for directing (98) the drop or a portion of the drop selectively towards a classification area (94, 96),
    - the decision for classifying the drop (6) or a portion of the drop, the decision consisting of selectively selecting a classification area from among the plurality of classification areas (94, 96),
    - the transport of the drop (6) respectively of a portion of the drop, towards the classification area of the drop (6) selected during the decision step.

11. The method according to any of the preceding claims, wherein at least one drop comprises at least one target element (37), at least one first signaling entity (34) capable of forming a complex with the target element (37) and at least a distinct second signaling entity (34) capable of forming a complex with the target element (37), the method comprising the measurement of a signal indicating the concentration of each of the signaling entities (34) re-localized on the aggregate (10).

12. The method according to any of the preceding claims, wherein at least one drop comprises at least one target element (37), at least one signaling entity (34) capable of forming a complex with the target element (37) and at least one quantification entity capable of forming a complex with the target element (37), the method comprising:

    - the measurement of a signal representative of the concentration of the signaling entity (34) re-localized on the aggregate (10),
    - the measurement of a signal representative of the concentration of the quantification entity re-localized on the aggregate (10),
    - the determination of the dissociation constant of the target element (37) with the signaling entity (34) from the ratio of the signaling entity signal (34) re-localized over the signal of the re-localized quantification entity.

13. The method according to claim 12, wherein at least certain drops (6) comprise a productive entity (90), the productive entity (90) being a cell which may produce at least one antibody being a target element (37), the method comprising a step for determining the affinity of the antibody produced by the productive entity (90) for at least one antigen, the method preferably comprising a step for sorting out the drop after the determination step.

14. The method according to any of the preceding claims, wherein at least one drop comprises at least two distinct signaling entities (34), each of both signaling entities (34) being capable of forming a complex with a distinct target element (37) on the aggregate (10), the method comprising the measurement of a signal indicating the concentration of each of the re-localized signaling entities (34).

15. The method according to claim 14, wherein at least certain drops (6) comprise a productive entity (90), the productive entity (90) being a cell which may produce one or several types of proteins, each protein being a distinct target element (37), the measurement of the signal indicating the concentration of each of the re-localized signaling entities (34) allowing quantification of the type(s) of proteins.

16. An apparatus for analyzing the content of drops comprising:

- an assembly for providing a plurality of drops (6) contained in a carrier fluid (8), at least one of the drops (6) comprising at least one aggregate (10) of particles (12) defining an elongated object along a main axis (X),

**characterized in that** the aggregate (10) of particles (12) comprises a column oriented along the main axis (X) and **in that** the apparatus comprises an assembly for measuring a physical parameter characteristic of the binding of a target element (37) on the aggregate (10), the assembly for measuring being adapted to measure the physical parameter locally in a plurality of points located in the drop (6), the measurement step preferably including the determination of the integral of the measured values within the drop (6), the apparatus further comprising preferably:

- an assembly for circulating the drop (22),
- an assembly for deciding classification of the drop,
- an assembly for sorting out the drop according to the classification decision.

FIG.1

Intensité de fluorescence

Temps

FIG.2

FIG.3

FIG.4

**FIG.5**

**FIG.6**

FIG.7

FIG.8

FIG.9

EP 3 207 373 B1

FIG.10

FIG.11

FIG.12

FIG.13

FIG.14

FIG.15

FIG.16

FIG.17

FIG.18

FIG.19

FIG.20

**FIG.21**

**FIG.22**

FIG.23

FIG.24

FIG.25

## FIG.26

## FIG.27

EP 3 207 373 B1

FIG.28

FIG.29

## FIG.30

## FIG.31

FIG.32

EP 3 207 373 B1

## FIG.33

## FIG.34

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2013041983 A1
- WO 2009011808 A1
- US 20100105112 A
- FR 2009051396 W

**Littérature non-brevet citée dans la description**

- **DE MAZUTIS et al.** Single-cell analysis and sorting using droplet-based microfluidics. *Nature Protocols illustre ce principe,* 04 Avril 2013
- **MAZUTIS et al.** *Nat prot,* 2013
- **BRANDON J DEKOSKY et al.** High-throughput sequencing of the paired human immunoglobulin heavy and light chain repertoire. *Nature Biotechnology,* 2013, vol. 31, 166-169